# EUROPEAN PATENT APPLICATION

(11) **EP 3 646 885 A1**
(43) Date of publication of application: **06.05.2020**
(21) Application number: 18825437.9
(22) Date of filing: 29.06.2018
(51) Int. Cl.: A61K 39/395, A61P 19/10, C07K 16/18, C07K 16/46

(54) **PEDIATRIC OSTEOPOROSIS DRUG THAT DOES NOT CAUSE GROWTH DISORDER**

(30) Priority: 30.06.2017 JP 2017129129
(71) Applicant: National University Corporation Hokkaido University, Sapporo-shi, Hokkaido 060-0808 (JP); Daiichi Sankyo Company, Limited, Tokyo 103-8426 (JP)
(72) Inventor: TAKAHATA, Masahiko, Sapporo-shi Hokkaido 060-0808 (JP); SATO, Dai, Sapporo-shi Hokkaido 060-0808 (JP); OTA, Masahiro, Sapporo-shi Hokkaido 060-0808 (JP); SHIMIZU, Tomohiro, Sapporo-shi Hokkaido 060-0808 (JP); FUKUDA, Chie, Tokyo 103-8426 (JP); TSUDA, Eisuke, Tokyo 103-8426 (JP)
(74) Representative: Wallace, Sheila Jane
(86) International application number: PCT/JP2018/025617
(87) International publication number: WO 2019/004487

(57) **Abstract**

An object of the present invention is to provide a pharmaceutical agent for the treatment and/or prophylaxis of pediatric osteoporosis without causing bone growth disorder in a subject to be medicated. A pharmaceutical composition for the treatment and/or prophylaxis of pediatric osteoporosis contains an antibody or a functional fragment thereof which binds to Siglec-15 and has activity of suppressing formation of osteoclasts and/or bone resorption by osteoclasts.

SEQUENCE LISTING

<110>  National University Corporation Hokkaido University
       DAIICHI SANKYO COMPANY, LIMITED

<120>  A drug for childhood osteoporosisthe osteoclast without growth
       disorder

<130>  PD20-9022WO

<150> JP 2017-129129

<151> 2017-6-30

<160>  22

<170>  PatentIn version 3.4

<210>  1
<211>  987
<212>  DNA
<213>  Homo sapiens


<220>
<221>  CDS
<222>  (1)..(987)

<400>  1
atg gaa aag tcc atc tgg ctg ctg gcc tgc ttg gcg tgg gtt ctc ccg        48
Met Glu Lys Ser Ile Trp Leu Leu Ala Cys Leu Ala Trp Val Leu Pro
1               5                   10                  15

aca ggc tca ttt gtg aga act aaa ata gat act acg gag aac ttg ctc        96
Thr Gly Ser Phe Val Arg Thr Lys Ile Asp Thr Thr Glu Asn Leu Leu
            20                  25                  30

aac aca gag gtg cac agc tcg cca gcg cag cgc tgg tcc atg cag gtg       144
Asn Thr Glu Val His Ser Ser Pro Ala Gln Arg Trp Ser Met Gln Val
        35                  40                  45

cca ccc gag gtg agc gcg gag gca ggc gac gcg gca gtg ctg ccc tgc       192
Pro Pro Glu Val Ser Ala Glu Ala Gly Asp Ala Ala Val Leu Pro Cys
    50                  55                  60

acc ttc acg cac ccg cac cgc cac tac gac ggg ccg ctg acg gcc atc       240
Thr Phe Thr His Pro His Arg His Tyr Asp Gly Pro Leu Thr Ala Ile
65                  70                  75                  80

tgg cgc gcg ggc gag ccc tat gcg ggc ccg cag gtg ttc cgc tgc gct       288
Trp Arg Ala Gly Glu Pro Tyr Ala Gly Pro Gln Val Phe Arg Cys Ala
                85                  90                  95

gcg gcg cgg ggc agc gag ctc tgc cag acg gcg ctg agc ctg cac ggc       336
Ala Ala Arg Gly Ser Glu Leu Cys Gln Thr Ala Leu Ser Leu His Gly
            100                 105                 110

cgc ttc cgg ctg ctg ggc aac ccg cgc cgc aac gac ctc tcg ctg cgc       384
Arg Phe Arg Leu Leu Gly Asn Pro Arg Arg Asn Asp Leu Ser Leu Arg
        115                 120                 125

gtc gag cgc ctc gcc ctg gct gac gac cgc cgc tac ttc tgc cgc gtc       432
Val Glu Arg Leu Ala Leu Ala Asp Asp Arg Arg Tyr Phe Cys Arg Val

130                 135                 140

gag ttc gcc ggc gac gtc cat gac cgc tac gag agc cgc cac ggc gtc       480
Glu Phe Ala Gly Asp Val His Asp Arg Tyr Glu Ser Arg His Gly Val
145                 150                 155                 160

cgg ctg cac gtg aca gcc gcg ccg cgg atc gtc aac atc tcg gtg ctg       528
Arg Leu His Val Thr Ala Ala Pro Arg Ile Val Asn Ile Ser Val Leu
                165                 170                 175

ccc agt ccg gct cac gcc ttc cgc gcg ctc tgc act gcc gaa ggg gag       576
Pro Ser Pro Ala His Ala Phe Arg Ala Leu Cys Thr Ala Glu Gly Glu
            180                 185                 190

ccg ccg ccc gcc ctc gcc tgg tcc ggc ccg gcc ctg ggc aac agc ttg       624
Pro Pro Pro Ala Leu Ala Trp Ser Gly Pro Ala Leu Gly Asn Ser Leu
        195                 200                 205

gca gcc gtg cgg agc ccg cgt gag ggt cac ggc cac cta gtg acc gcc       672
Ala Ala Val Arg Ser Pro Arg Glu Gly His Gly His Leu Val Thr Ala
    210                 215                 220

gaa ctg ccc gca ctg acc cat gac ggc cgc tac acg tgt acg gcc gcc       720
Glu Leu Pro Ala Leu Thr His Asp Gly Arg Tyr Thr Cys Thr Ala Ala
225                 230                 235                 240

aac agc ctg ggc cgc tcc gag gcc agc gtc tac ctg ttc cgc ttc cat       768
Asn Ser Leu Gly Arg Ser Glu Ala Ser Val Tyr Leu Phe Arg Phe His
                245                 250                 255

ggc gcc agc ggg gcc tcg acg gtc gcc ctc ctg ctc ggc gct ctc ggc       816
Gly Ala Ser Gly Ala Ser Thr Val Ala Leu Leu Leu Gly Ala Leu Gly
            260                 265                 270

ttc aag gcg ctg ctg ctg ctc ggg gtc ctg gcc gcc cgc gct gcc cgc       864
Phe Lys Ala Leu Leu Leu Leu Gly Val Leu Ala Ala Arg Ala Ala Arg
        275                 280                 285

cgc cgc cca gag cat ctg gac acc ccg gac acc cca cca cgg tcc cag       912
Arg Arg Pro Glu His Leu Asp Thr Pro Asp Thr Pro Pro Arg Ser Gln
    290                 295                 300

gcc cag gag tcc aat tat gaa aat ttg agc cag atg aac ccc cgg agc       960
Ala Gln Glu Ser Asn Tyr Glu Asn Leu Ser Gln Met Asn Pro Arg Ser
305                 310                 315                 320

cca cca gcc acc atg tgc tca ccg tga                                   987
Pro Pro Ala Thr Met Cys Ser Pro
                325

<210>  2
<211>  328
<212>  PRT
<213>  Homo sapiens

<400>  2

Met Glu Lys Ser Ile Trp Leu Leu Ala Cys Leu Ala Trp Val Leu Pro
1               5                   10                  15

Thr Gly Ser Phe Val Arg Thr Lys Ile Asp Thr Thr Glu Asn Leu Leu

20                  25                  30

Asn Thr Glu Val His Ser Ser Pro Ala Gln Arg Trp Ser Met Gln Val
        35                  40                  45

Pro Pro Glu Val Ser Ala Glu Ala Gly Asp Ala Ala Val Leu Pro Cys
    50                  55                  60

Thr Phe Thr His Pro His Arg His Tyr Asp Gly Pro Leu Thr Ala Ile
65                  70                  75                  80

Trp Arg Ala Gly Glu Pro Tyr Ala Gly Pro Gln Val Phe Arg Cys Ala
                85                  90                  95

Ala Ala Arg Gly Ser Glu Leu Cys Gln Thr Ala Leu Ser Leu His Gly
            100                 105                 110

Arg Phe Arg Leu Leu Gly Asn Pro Arg Arg Asn Asp Leu Ser Leu Arg
        115                 120                 125

Val Glu Arg Leu Ala Leu Ala Asp Asp Arg Arg Tyr Phe Cys Arg Val
    130                 135                 140

Glu Phe Ala Gly Asp Val His Asp Arg Tyr Glu Ser Arg His Gly Val
145                 150                 155                 160

Arg Leu His Val Thr Ala Ala Pro Arg Ile Val Asn Ile Ser Val Leu
                165                 170                 175

Pro Ser Pro Ala His Ala Phe Arg Ala Leu Cys Thr Ala Glu Gly Glu
            180                 185                 190

Pro Pro Pro Ala Leu Ala Trp Ser Gly Pro Ala Leu Gly Asn Ser Leu
        195                 200                 205

Ala Ala Val Arg Ser Pro Arg Glu Gly His Gly His Leu Val Thr Ala
    210                 215                 220

Glu Leu Pro Ala Leu Thr His Asp Gly Arg Tyr Thr Cys Thr Ala Ala
225                 230                 235                 240

Asn Ser Leu Gly Arg Ser Glu Ala Ser Val Tyr Leu Phe Arg Phe His
                245                 250                 255

Gly Ala Ser Gly Ala Ser Thr Val Ala Leu Leu Leu Gly Ala Leu Gly
            260                 265                 270

Phe Lys Ala Leu Leu Leu Leu Gly Val Leu Ala Ala Arg Ala Ala Arg
        275                 280                 285

Arg Arg Pro Glu His Leu Asp Thr Pro Asp Thr Pro Pro Arg Ser Gln
    290                 295                 300

Ala Gln Glu Ser Asn Tyr Glu Asn Leu Ser Gln Met Asn Pro Arg Ser
305                 310                 315                 320

Pro Pro Ala Thr Met Cys Ser Pro
                325

<210>  3
<211>  1026
<212>  DNA
<213>  Mus musculus

<220>
<221>  CDS
<222>  (1)..(1026)

<400>  3
atg gag ggg tcc ctc caa ctc ctg gcc tgc ttg gcc tgt gtg ctc cag        48
Met Glu Gly Ser Leu Gln Leu Leu Ala Cys Leu Ala Cys Val Leu Gln
1               5                   10                  15

atg gga tcc ctt gtg aaa act aga aga gac gct tcg ggg gat ctg ctc        96
Met Gly Ser Leu Val Lys Thr Arg Arg Asp Ala Ser Gly Asp Leu Leu
            20                  25                  30

aac aca gag gcg cac agt gcc ccg gcg cag cgc tgg tcc atg cag gtg       144
Asn Thr Glu Ala His Ser Ala Pro Ala Gln Arg Trp Ser Met Gln Val
        35                  40                  45

ccc gcg gag gtg aac gcg gag gct ggc gac gcg gcg gtg ctg ccc tgc       192
Pro Ala Glu Val Asn Ala Glu Ala Gly Asp Ala Ala Val Leu Pro Cys
    50                  55                  60

acc ttc acg cac ccg cac cgc cac tac gac ggg ccg ctg acg gcc atc       240
Thr Phe Thr His Pro His Arg His Tyr Asp Gly Pro Leu Thr Ala Ile
65                  70                  75                  80

tgg cgc tcg ggc gag ccg tac gcg ggc ccg cag gtg ttc cgc tgc acc       288
Trp Arg Ser Gly Glu Pro Tyr Ala Gly Pro Gln Val Phe Arg Cys Thr
                85                  90                  95

gcg gcg ccg ggc agc gag ctg tgc cag acg gcg ctg agc ctg cac ggc       336
Ala Ala Pro Gly Ser Glu Leu Cys Gln Thr Ala Leu Ser Leu His Gly
            100                 105                 110

cgc ttc cgc ctg ctg ggc aac ccg cgc cgc aac gac ctg tcc ctg cgc       384
Arg Phe Arg Leu Leu Gly Asn Pro Arg Arg Asn Asp Leu Ser Leu Arg
        115                 120                 125

gtc gag cgc ctc gcc ctg gcg gac agc ggc cgc tac ttc tgc cgc gtg       432
Val Glu Arg Leu Ala Leu Ala Asp Ser Gly Arg Tyr Phe Cys Arg Val
    130                 135                 140

gag ttc acc ggc gac gcc cac gat cgc tat gag agt cgc cat ggg gtc 480
Glu Phe Thr Gly Asp Ala His Asp Arg Tyr Glu Ser Arg His Gly Val
145                 150                 155                 160

cgt ctg cgc gtg act gct gcg ccg cgg atc gtc aac atc tcg gtg ctg 528
Arg Leu Arg Val Thr Ala Ala Pro Arg Ile Val Asn Ile Ser Val Leu
                165                 170                 175

ccg ggc ccc gcg cac gcc ttc cgc gcg ctc tgc acc gcc gag ggg gag 576
Pro Gly Pro Ala His Ala Phe Arg Ala Leu Cys Thr Ala Glu Gly Glu
            180                 185                 190

ccc ccg ccc gcc ctc gcc tgg tcg ggt ccc gcc cca ggc aac agc tcc 624
Pro Pro Pro Ala Leu Ala Trp Ser Gly Pro Ala Pro Gly Asn Ser Ser
        195                 200                 205

gct gcc ctg cag ggc cag ggt cac ggc tac cag gtg acc gcc gag ttg 672
Ala Ala Leu Gln Gly Gln Gly His Gly Tyr Gln Val Thr Ala Glu Leu
    210                 215                 220

ccc gcg ctg acc cgc gac ggc cgc tac acg tgc acg gcg gcc aat agc 720
Pro Ala Leu Thr Arg Asp Gly Arg Tyr Thr Cys Thr Ala Ala Asn Ser
225                 230                 235                 240

ctg ggc cgc gcc gag gcc agc gtc tac ctg ttc cgc ttc cac ggc gcc 768
Leu Gly Arg Ala Glu Ala Ser Val Tyr Leu Phe Arg Phe His Gly Ala
                245                 250                 255

ccc gga acc tcg acc cta gcg ctc ctg ctg ggc gcg ctg ggc ctc aag 816
Pro Gly Thr Ser Thr Leu Ala Leu Leu Leu Gly Ala Leu Gly Leu Lys
            260                 265                 270

gcc ttg ctg ctg ctt ggc att ctg gga gcg cgt gcc acc cga cgc cga 864
Ala Leu Leu Leu Leu Gly Ile Leu Gly Ala Arg Ala Thr Arg Arg Arg
        275                 280                 285

cta gat cac ctg gtc ccc cag gac acc cct cca cgg tct cag gct cag 912
Leu Asp His Leu Val Pro Gln Asp Thr Pro Pro Arg Ser Gln Ala Gln
    290                 295                 300

gag tcc aat tat gaa aat ttg agc cag atg agt cct cca ggc cac cag 960
Glu Ser Asn Tyr Glu Asn Leu Ser Gln Met Ser Pro Pro Gly His Gln
305                 310                 315                 320

ctg cca cgt gtt tgc tgt gag gaa ctc ctc agc cat cac cat cta gtc 1008
Leu Pro Arg Val Cys Cys Glu Glu Leu Leu Ser His His His Leu Val
                325                 330                 335

att cac cat gag aaa taa 1026
Ile His His Glu Lys
            340

<210> 4
<211> 341
<212> PRT
<213> Mus musculus

<400> 4

Met Glu Gly Ser Leu Gln Leu Leu Ala Cys Leu Ala Cys Val Leu Gln
1               5                   10                  15

Met Gly Ser Leu Val Lys Thr Arg Arg Asp Ala Ser Gly Asp Leu Leu
            20                  25                  30

Asn Thr Glu Ala His Ser Ala Pro Ala Gln Arg Trp Ser Met Gln Val
        35                  40                  45

Pro Ala Glu Val Asn Ala Glu Ala Gly Asp Ala Ala Val Leu Pro Cys
    50                  55                  60

Thr Phe Thr His Pro His Arg His Tyr Asp Gly Pro Leu Thr Ala Ile
65                  70                  75                  80

Trp Arg Ser Gly Glu Pro Tyr Ala Gly Pro Gln Val Phe Arg Cys Thr
                85                  90                  95

Ala Ala Pro Gly Ser Glu Leu Cys Gln Thr Ala Leu Ser Leu His Gly
            100                 105                 110

Arg Phe Arg Leu Leu Gly Asn Pro Arg Arg Asn Asp Leu Ser Leu Arg
        115                 120                 125

Val Glu Arg Leu Ala Leu Ala Asp Ser Gly Arg Tyr Phe Cys Arg Val
    130                 135                 140

Glu Phe Thr Gly Asp Ala His Asp Arg Tyr Glu Ser Arg His Gly Val
145                 150                 155                 160

Arg Leu Arg Val Thr Ala Ala Pro Arg Ile Val Asn Ile Ser Val Leu
                165                 170                 175

Pro Gly Pro Ala His Ala Phe Arg Ala Leu Cys Thr Ala Glu Gly Glu
            180                 185                 190

Pro Pro Pro Ala Leu Ala Trp Ser Gly Pro Ala Pro Gly Asn Ser Ser
        195                 200                 205

Ala Ala Leu Gln Gly Gln Gly His Gly Tyr Gln Val Thr Ala Glu Leu
    210                 215                 220

Pro Ala Leu Thr Arg Asp Gly Arg Tyr Thr Cys Thr Ala Ala Asn Ser
225                 230                 235                 240

Leu Gly Arg Ala Glu Ala Ser Val Tyr Leu Phe Arg Phe His Gly Ala
                245                 250                 255

Pro Gly Thr Ser Thr Leu Ala Leu Leu Leu Gly Ala Leu Gly Leu Lys
            260                 265                 270

Ala Leu Leu Leu Leu Gly Ile Leu Gly Ala Arg Ala Thr Arg Arg Arg
        275                 280                 285

Leu Asp His Leu Val Pro Gln Asp Thr Pro Pro Arg Ser Gln Ala Gln
    290                 295                 300

Glu Ser Asn Tyr Glu Asn Leu Ser Gln Met Ser Pro Pro Gly His Gln
305                 310                 315                 320

Leu Pro Arg Val Cys Cys Glu Glu Leu Leu Ser His His His Leu Val
                325                 330                 335

Ile His His Glu Lys
            340

<210>  5
<211>  774
<212>  DNA
<213>  Mus musculus

<220>
<221>  CDS
<222>  (1)..(774)

<400>  5
atg gag ggg tcc ctc caa ctc ctg gcc tgc ttg gcc tgt gtg ctc cag      48
Met Glu Gly Ser Leu Gln Leu Leu Ala Cys Leu Ala Cys Val Leu Gln
1               5                   10                  15

atg gga tcc ctt gtg aaa act aga aga gac gct tcg ggg gat ctg ctc      96
Met Gly Ser Leu Val Lys Thr Arg Arg Asp Ala Ser Gly Asp Leu Leu
            20                  25                  30

aac aca gag gcg cac agt gcc ccg gcg cag cgc tgg tcc atg cag gtg      144
Asn Thr Glu Ala His Ser Ala Pro Ala Gln Arg Trp Ser Met Gln Val
        35                  40                  45

ccc gcg gag gtg aac gcg gag gct ggc gac gcg gcg gtg ctg ccc tgc      192
Pro Ala Glu Val Asn Ala Glu Ala Gly Asp Ala Ala Val Leu Pro Cys
    50                  55                  60

acc ttc acg cac ccg cac cgc cac tac gac ggg ccg ctg acg gcc atc      240
Thr Phe Thr His Pro His Arg His Tyr Asp Gly Pro Leu Thr Ala Ile
65                  70                  75                  80

tgg cgc tcg ggc gag ccg tac gcg ggc ccg cag gtg ttc cgc tgc acc      288
Trp Arg Ser Gly Glu Pro Tyr Ala Gly Pro Gln Val Phe Arg Cys Thr
                85                  90                  95

gcg gcg ccg ggc agc gag ctg tgc cag acg gcg ctg agc ctg cac ggc      336
Ala Ala Pro Gly Ser Glu Leu Cys Gln Thr Ala Leu Ser Leu His Gly
            100                 105                 110

cgc ttc cgc ctg ctg ggc aac ccg cgc cgc aac gac ctg tcc ctg cgc      384
Arg Phe Arg Leu Leu Gly Asn Pro Arg Arg Asn Asp Leu Ser Leu Arg

115                 120                 125

gtc gag cgc ctc gcc ctg gcg gac agc ggc cgc tac ttc tgc cgc gtg      432
Val Glu Arg Leu Ala Leu Ala Asp Ser Gly Arg Tyr Phe Cys Arg Val
    130                 135                 140

gag ttc acc ggc gac gcc cac gat cgc tat gag agt cgc cat ggg gtc      480
Glu Phe Thr Gly Asp Ala His Asp Arg Tyr Glu Ser Arg His Gly Val
145                 150                 155                 160

cgt ctg cgc gtg act gct gcg ccg cgg atc gtc aac atc tcg gtg ctg      528
Arg Leu Arg Val Thr Ala Ala Pro Arg Ile Val Asn Ile Ser Val Leu
                165                 170                 175

ccg ggc ccc gcg cac gcc ttc cgc gcg ctc tgc acc gcc gag ggg gag      576
Pro Gly Pro Ala His Ala Phe Arg Ala Leu Cys Thr Ala Glu Gly Glu
            180                 185                 190

ccc ccg ccc gcc ctc gcc tgg tcg ggt ccc gcc cca ggc aac agc tcc      624
Pro Pro Pro Ala Leu Ala Trp Ser Gly Pro Ala Pro Gly Asn Ser Ser
        195                 200                 205

gct gcc ctg cag ggc cag ggt cac ggc tac cag gtg acc gcc gag ttg      672
Ala Ala Leu Gln Gly Gln Gly His Gly Tyr Gln Val Thr Ala Glu Leu
    210                 215                 220

ccc gcg ctg acc cgc gac ggc cgc tac acg tgc acg gcg gcc aat agc      720
Pro Ala Leu Thr Arg Asp Gly Arg Tyr Thr Cys Thr Ala Ala Asn Ser
225                 230                 235                 240

ctg ggc cgc gcc gag gcc agc gtc tac ctg ttc cgc ttc cac ggc gcc      768
Leu Gly Arg Ala Glu Ala Ser Val Tyr Leu Phe Arg Phe His Gly Ala
                245                 250                 255

ccc gga                                                              774
Pro Gly



<210>  6
<211>  780
<212>  DNA
<213>  Homo sapiens



<220>
<221>  CDR
<222>  (1)..(780)

<220>
<221>  CDS
<222>  (1)..(780)

<400>  6
atg gaa aag tcc atc tgg ctg ctg gcc tgc ttg gcg tgg gtt ctc ccg       48
Met Glu Lys Ser Ile Trp Leu Leu Ala Cys Leu Ala Trp Val Leu Pro
1               5                   10                  15

aca ggc tca ttt gtg aga act aaa ata gat act acg gag aac ttg ctc       96
Thr Gly Ser Phe Val Arg Thr Lys Ile Asp Thr Thr Glu Asn Leu Leu
            20                  25                  30

aac aca gag gtg cac agc tcg cca gcg cag cgc tgg tcc atg cag gtg      144

Asn Thr Glu Val His Ser Ser Pro Ala Gln Arg Trp Ser Met Gln Val
        35                  40                  45

cca ccc gag gtg agc gcg gag gca ggc gac gcg gca gtg ctg ccc tgc 192
Pro Pro Glu Val Ser Ala Glu Ala Gly Asp Ala Ala Val Leu Pro Cys
    50                  55                  60

acc ttc acg cac ccg cac cgc cac tac gac ggg ccg ctg acg gcc atc 240
Thr Phe Thr His Pro His Arg His Tyr Asp Gly Pro Leu Thr Ala Ile
65                  70                  75                  80

tgg cgc gcg ggc gag ccc tat gcg ggc ccg cag gtg ttc cgc tgc gct 288
Trp Arg Ala Gly Glu Pro Tyr Ala Gly Pro Gln Val Phe Arg Cys Ala
                85                  90                  95

gcg gcg cgg ggc agc gag ctc tgc cag acg gcg ctg agc ctg cac ggc 336
Ala Ala Arg Gly Ser Glu Leu Cys Gln Thr Ala Leu Ser Leu His Gly
            100                 105                 110

cgc ttc cgg ctg ctg ggc aac ccg cgc cgc aac gac ctc tcg ctg cgc 384
Arg Phe Arg Leu Leu Gly Asn Pro Arg Arg Asn Asp Leu Ser Leu Arg
        115                 120                 125

gtc gag cgc ctc gcc ctg gct gac gac cgc cgc tac ttc tgc cgc gtc 432
Val Glu Arg Leu Ala Leu Ala Asp Asp Arg Arg Tyr Phe Cys Arg Val
    130                 135                 140

gag ttc gcc ggc gac gtc cat gac cgc tac gag agc cgc cac ggc gtc 480
Glu Phe Ala Gly Asp Val His Asp Arg Tyr Glu Ser Arg His Gly Val
145                 150                 155                 160

cgg ctg cac gtg aca gcc gcg ccg cgg atc gtc aac atc tcg gtg ctg 528
Arg Leu His Val Thr Ala Ala Pro Arg Ile Val Asn Ile Ser Val Leu
                165                 170                 175

ccc agt ccg gct cac gcc ttc cgc gcg ctc tgc act gcc gaa ggg gag 576
Pro Ser Pro Ala His Ala Phe Arg Ala Leu Cys Thr Ala Glu Gly Glu
            180                 185                 190

ccg ccg ccc gcc ctc gcc tgg tcc ggc ccg gcc ctg ggc aac agc ttg 624
Pro Pro Pro Ala Leu Ala Trp Ser Gly Pro Ala Leu Gly Asn Ser Leu
        195                 200                 205

gca gcc gtg cgg agc ccg cgt gag ggt cac ggc cac cta gtg acc gcc 672
Ala Ala Val Arg Ser Pro Arg Glu Gly His Gly His Leu Val Thr Ala
    210                 215                 220

gaa ctg ccc gca ctg acc cat gac ggc cgc tac acg tgt acg gcc gcc 720
Glu Leu Pro Ala Leu Thr His Asp Gly Arg Tyr Thr Cys Thr Ala Ala
225                 230                 235                 240

aac agc ctg ggc cgc tcc gag gcc agc gtc tac ctg ttc cgc ttc cat 768
Asn Ser Leu Gly Arg Ser Glu Ala Ser Val Tyr Leu Phe Arg Phe His
                245                 250                 255

ggc gcc agc ggg 780
Gly Ala Ser Gly
            260

<210> 7
<211> 466
<212> PRT

<213>  Artificial Sequence

<220>
<223>  Synthetic Construct

<400>  7

Met Lys His Leu Trp Phe Phe Leu Leu Leu Val Ala Ala Pro Arg Trp
1               5                   10                  15

Val Leu Ser Glu Val Gln Val Val Glu Ser Gly Gly Gly Leu Val Gln
            20                  25                  30

Pro Gly Gly Ser Leu Arg Leu Ser Cys Ala Thr Ser Gly Phe Asn Phe
        35                  40                  45

Asn Asp Tyr Phe Met Asn Trp Val Arg Gln Ala Pro Gly Lys Gly Leu
    50                  55                  60

Glu Trp Val Ala Gln Ile Arg Asn Lys Ile Tyr Thr Tyr Ala Thr Phe
65                  70                  75                  80

Tyr Ala Glu Ser Leu Glu Gly Arg Phe Thr Ile Ser Arg Asp Asn Ser
                85                  90                  95

Lys Ser Thr Val Tyr Leu Gln Met Asn Ser Leu Arg Ala Glu Asp Thr
            100                 105                 110

Ala Val Tyr Tyr Cys Thr Arg Ser Leu Thr Gly Gly Asp Tyr Phe Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  8
<211>  238
<212>  PRT
<213>  Artificial Sequence

<220>
<223>  Synthetic Construct

<400>  8

Met Val Leu Gln Thr Gln Val Phe Ile Ser Leu Leu Leu Trp Ile Ser
1               5                   10                  15

Gly Ala Tyr Gly Asp Ile Val Leu Thr Gln Ser Pro Asp Ser Leu Ala
            20                  25                  30

Val Ser Leu Gly Glu Arg Ala Thr Ile Asn Cys Arg Ala Ser Gln Ser
        35                  40                  45

Val Thr Ile Ser Gly Tyr Ser Phe Ile His Trp Tyr Gln Gln Lys Pro
    50                  55                  60

Gly Gln Pro Pro Lys Leu Leu Ile Tyr Arg Ala Ser Asn Leu Ala Ser
65                  70                  75                  80

Gly Ile Pro Ala Arg Phe Ser Gly Ser Gly Ser Gly Thr Asp Phe Thr
                85                  90                  95

Leu Thr Ile Ser Ser Leu Gln Ala Glu Asp Val Ala Thr Tyr Tyr Cys
            100                 105                 110

Gln Gln Ser Arg Lys Ser Pro Trp Thr Phe Gly Gln Gly Thr Lys Val
        115                 120                 125

Glu Ile Lys Arg Thr Val Ala Ala Pro Ser Val Phe Ile Phe Pro Pro
    130                 135                 140

Ser Asp Glu Gln Leu Lys Ser Gly Thr Ala Ser Val Val Cys Leu Leu
145                 150                 155                 160

Asn Asn Phe Tyr Pro Arg Glu Ala Lys Val Gln Trp Lys Val Asp Asn
                165                 170                 175

Ala Leu Gln Ser Gly Asn Ser Gln Glu Ser Val Thr Glu Gln Asp Ser
            180                 185                 190

Lys Asp Ser Thr Tyr Ser Leu Ser Ser Thr Leu Thr Leu Ser Lys Ala
        195                 200                 205

Asp Tyr Glu Lys His Lys Val Tyr Ala Cys Glu Val Thr His Gln Gly
    210                 215                 220

Leu Ser Ser Pro Val Thr Lys Ser Phe Asn Arg Gly Glu Cys
225                 230                 235

<210>  9
<211>  466
<212>  PRT
<213>  Artificial Sequence

<220>
<223>  Synthetic Construct

<400>  9

Met Lys His Leu Trp Phe Phe Leu Leu Leu Val Ala Ala Pro Arg Trp
1               5                   10                  15

Val Leu Ser Glu Val Gln Leu Val Glu Ser Gly Gly Gly Leu Val Gln
            20                  25                  30

Pro Gly Gly Ser Leu Arg Leu Ser Cys Ala Ala Ser Gly Phe Asn Phe
        35                  40                  45

Asn Asp Tyr Phe Met Asn Trp Val Arg Gln Ala Pro Gly Lys Gly Leu
    50                  55                  60

Glu Trp Val Ala Gln Ile Arg Asn Lys Ile Tyr Thr Tyr Ala Thr Phe
65                  70                  75                  80

Tyr Ala Ala Ser Val Lys Gly Arg Phe Thr Ile Ser Arg Asp Asn Ala
                85                  90                  95

Lys Asn Ser Leu Tyr Leu Gln Met Asn Ser Leu Arg Ala Glu Asp Thr
            100                 105                 110

Ala Val Tyr Tyr Cys Ala Arg Ser Leu Thr Gly Gly Asp Tyr Phe Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr

            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  10
<211>  238
<212>  PRT
<213>  Artificial Sequence

<220>
<223>  Synthetic Construct

<400>  10

Met Val Leu Gln Thr Gln Val Phe Ile Ser Leu Leu Leu Trp Ile Ser
1               5                   10                  15

Gly Ala Tyr Gly Glu Ile Leu Met Thr Gln Ser Pro Ala Thr Leu Ser
            20                  25                  30

Leu Ser Pro Gly Glu Arg Ala Thr Leu Ser Cys Arg Ala Ser Gln Ser
        35                  40                  45

Val Thr Ile Ser Gly Tyr Ser Phe Ile His Trp Tyr Gln Gln Lys Pro
    50                  55                  60

Gly Gln Ala Pro Arg Leu Leu Ile Tyr Arg Ala Ser Asn Leu Ala Ser
65                  70                  75                  80

Gly Ile Pro Ala Arg Phe Ser Gly Ser Gly Ser Gly Thr Asp Phe Thr
                85                  90                  95

Leu Thr Ile Ser Ser Leu Glu Pro Glu Asp Phe Ala Leu Tyr Tyr Cys
            100                 105                 110

Gln Gln Ser Arg Lys Ser Pro Trp Thr Phe Gly Gln Gly Thr Lys Val
        115                 120                 125

Glu Ile Lys Arg Thr Val Ala Ala Pro Ser Val Phe Ile Phe Pro Pro
    130                 135                 140

Ser Asp Glu Gln Leu Lys Ser Gly Thr Ala Ser Val Val Cys Leu Leu
145                 150                 155                 160

Asn Asn Phe Tyr Pro Arg Glu Ala Lys Val Gln Trp Lys Val Asp Asn

165                 170                 175

Ala Leu Gln Ser Gly Asn Ser Gln Glu Ser Val Thr Glu Gln Asp Ser
            180                 185                 190

Lys Asp Ser Thr Tyr Ser Leu Ser Ser Thr Leu Thr Leu Ser Lys Ala
        195                 200                 205

Asp Tyr Glu Lys His Lys Val Tyr Ala Cys Glu Val Thr His Gln Gly
    210                 215                 220

Leu Ser Ser Pro Val Thr Lys Ser Phe Asn Arg Gly Glu Cys
225                 230                 235

<210>  11
<211>  238
<212>  PRT
<213>  Artificial Sequence

<220>
<223>  Synthetic Construct

<400>  11

Met Val Leu Gln Thr Gln Val Phe Ile Ser Leu Leu Leu Trp Ile Ser
1               5                   10                  15

Gly Ala Tyr Gly Glu Ile Leu Met Thr Gln Ser Pro Ala Thr Leu Ser
            20                  25                  30

Leu Ser Pro Gly Glu Arg Ala Thr Leu Ser Cys Arg Ala Ser Gln Ser
        35                  40                  45

Val Thr Ile Ser Gly Tyr Ser Phe Ile His Trp Tyr Gln Gln Lys Pro
    50                  55                  60

Gly Gln Ala Pro Arg Leu Leu Ile Tyr Arg Ala Ser Asn Leu Ala Ser
65                  70                  75                  80

Gly Ile Pro Ala Arg Phe Ser Gly Ser Gly Ser Gly Thr Asp Phe Thr
                85                  90                  95

Leu Thr Ile Ser Ser Leu Glu Pro Glu Asp Phe Ala Leu Tyr Phe Cys
            100                 105                 110

Gln Gln Ser Arg Lys Ser Pro Trp Thr Phe Ala Gln Gly Thr Lys Val
        115                 120                 125

Glu Ile Lys Arg Thr Val Ala Ala Pro Ser Val Phe Ile Phe Pro Pro
    130                 135                 140

Ser Asp Glu Gln Leu Lys Ser Gly Thr Ala Ser Val Val Cys Leu Leu
145                 150                 155                 160

Asn Asn Phe Tyr Pro Arg Glu Ala Lys Val Gln Trp Lys Val Asp Asn
                165                 170                 175

Ala Leu Gln Ser Gly Asn Ser Gln Glu Ser Val Thr Glu Gln Asp Ser
            180                 185                 190

Lys Asp Ser Thr Tyr Ser Leu Ser Ser Thr Leu Thr Leu Ser Lys Ala
        195                 200                 205

Asp Tyr Glu Lys His Lys Val Tyr Ala Cys Glu Val Thr His Gln Gly
    210                 215                 220

Leu Ser Ser Pro Val Thr Lys Ser Phe Asn Arg Gly Glu Cys
225                 230                 235

<210>  12
<211>  5
<212>  PRT
<213>  Rattus norvegicus

<400>  12

Asp Tyr Phe Met Asn
1               5

<210>  13
<211>  14
<212>  PRT
<213>  Rattus norvegicus

<400>  13

Gln Ile Arg Asn Lys Ile Tyr Thr Tyr Ala Thr Phe Tyr Ala
1               5                   10

<210>  14
<211>  10
<212>  PRT
<213>  Rattus norvegicus

<400>  14

Ser Leu Thr Gly Gly Asp Tyr Phe Asp Tyr
1               5                   10

<210>  15
<211>  15
<212>  PRT
<213>  Rattus norvegicus

<400>  15

Arg Ala Ser Gln Ser Val Thr Ile Ser Gly Tyr Ser Phe Ile His
1               5                   10                  15


<210>  16
<211>  7
<212>  PRT
<213>  Rattus norvegicus

<400>  16

Arg Ala Ser Asn Leu Ala Ser
1               5


<210>  17
<211>  9
<212>  PRT
<213>  Rattus norvegicus

<400>  17

Gln Gln Ser Arg Lys Ser Pro Trp Thr
1               5


<210>  18
<211>  10
<212>  PRT
<213>  Artificial Sequence

<220>
<223>  Synthetic Construct

<400>  18

Ser Leu Glu Gly Gly Asp Tyr Phe Asp Tyr
1               5                   10


<210>  19
<211>  466
<212>  PRT
<213>  Artificial Sequence

<220>
<223>  Synthetic Construct

<400>  19

Met Lys His Leu Trp Phe Phe Leu Leu Leu Val Ala Ala Pro Arg Trp
1               5                   10                  15


Val Leu Ser Glu Val Gln Leu Val Glu Ser Gly Gly Gly Leu Val Gln
            20                  25                  30


Pro Gly Gly Ser Leu Arg Leu Ser Cys Ala Ala Ser Gly Phe Asn Phe
        35                  40                  45

Asn Asp Tyr Phe Met Asn Trp Val Arg Gln Ala Pro Gly Lys Gly Leu
    50                  55                  60

Glu Trp Val Ala Gln Ile Arg Asn Lys Ile Tyr Thr Tyr Ala Thr Phe
65                  70                  75                  80

Tyr Ala Ala Ser Val Lys Gly Arg Phe Thr Ile Ser Arg Asp Asn Ala
                85                  90                  95

Lys Asn Ser Leu Tyr Leu Gln Met Asn Ser Leu Arg Ala Glu Asp Thr
            100                 105                 110

Ala Val Tyr Tyr Cys Ala Arg Ser Leu Glu Gly Gly Asp Tyr Phe Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Cys Ser Arg Ser Thr Ser Glu
145                 150                 155                 160

Ser Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Asn Phe Gly Thr Gln Thr Tyr Thr Cys Asn
    210                 215                 220

Val Asp His Lys Pro Ser Asn Thr Lys Val Asp Lys Thr Val Glu Arg
225                 230                 235                 240

Lys Cys Cys Val Glu Cys Pro Pro Cys Pro Ala Pro Pro Val Ala Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser His Glu
        275                 280                 285

Asp Pro Glu Val Gln Phe Asn Trp Tyr Val Asp Gly Val Glu Val His

290                 295                 300

Asn Ala Lys Thr Lys Pro Arg Glu Glu Gln Phe Asn Ser Thr Phe Arg
305                 310                 315                 320

Val Val Ser Val Leu Thr Val Val His Gln Asp Trp Leu Asn Gly Lys
                325                 330                 335

Glu Tyr Lys Cys Lys Val Ser Asn Lys Gly Leu Pro Ala Pro Ile Glu
            340                 345                 350

Lys Thr Ile Ser Lys Thr Lys Gly Gln Pro Arg Glu Pro Gln Val Tyr
        355                 360                 365

Thr Leu Pro Pro Ser Arg Glu Glu Met Thr Lys Asn Gln Val Ser Leu
    370                 375                 380

Thr Cys Leu Val Lys Gly Phe Tyr Pro Ser Asp Ile Ala Val Glu Trp
385                 390                 395                 400

Glu Ser Asn Gly Gln Pro Glu Asn Asn Tyr Lys Thr Thr Pro Pro Met
                405                 410                 415

Leu Asp Ser Asp Gly Ser Phe Phe Leu Tyr Ser Lys Leu Thr Val Asp
            420                 425                 430

Lys Ser Arg Trp Gln Gln Gly Asn Val Phe Ser Cys Ser Val Met His
        435                 440                 445

Glu Ala Leu His Asn His Tyr Thr Gln Lys Ser Leu Ser Leu Ser Pro
    450                 455                 460

Gly Lys
465

<210>  20
<211>  238
<212>  PRT
<213>  Artificial Sequence

<220>
<223>  Synthetic Construct

<400>  20

Met Val Leu Gln Thr Gln Val Phe Ile Ser Leu Leu Leu Trp Ile Ser
1               5                   10                  15

Gly Ala Tyr Gly Glu Ile Leu Met Thr Gln Ser Pro Ala Thr Leu Ser
            20                  25                  30

Leu Ser Pro Gly Glu Arg Ala Thr Leu Ser Cys Arg Ala Ser Gln Ser
        35                  40                  45

Val Thr Ile Ser Gly Tyr Ser Phe Ile His Trp Tyr Gln Gln Lys Pro
    50                  55                  60

Gly Gln Ala Pro Arg Leu Leu Ile Tyr Arg Ala Ser Asn Leu Ala Ser
65                  70                  75                  80

Gly Ile Pro Ala Arg Phe Ser Gly Ser Gly Ser Gly Thr Asp Phe Thr
                85                  90                  95

Leu Thr Ile Ser Ser Leu Glu Pro Glu Asp Phe Ala Leu Tyr Tyr Cys
            100                 105                 110

Gln Gln Ser Arg Lys Ser Pro Trp Thr Phe Gly Gln Gly Thr Lys Val
        115                 120                 125

Glu Ile Lys Arg Thr Val Ala Ala Pro Ser Val Phe Ile Phe Pro Pro
    130                 135                 140

Ser Asp Glu Gln Leu Lys Ser Gly Thr Ala Ser Val Val Cys Leu Leu
145                 150                 155                 160

Asn Asn Phe Tyr Pro Arg Glu Ala Lys Val Gln Trp Lys Val Asp Asn
                165                 170                 175

Ala Leu Gln Ser Gly Asn Ser Gln Glu Ser Val Thr Glu Gln Asp Ser
            180                 185                 190

Lys Asp Ser Thr Tyr Ser Leu Ser Ser Thr Leu Thr Leu Ser Lys Ala
        195                 200                 205

Asp Tyr Glu Lys His Lys Val Tyr Ala Cys Glu Val Thr His Gln Gly
    210                 215                 220

Leu Ser Ser Pro Val Thr Lys Ser Phe Asn Arg Gly Glu Cys
225                 230                 235

<210>  21
<211>  167
<212>  PRT
<213>  Rattus norvegicus

<400>  21

Met Lys Leu Trp Leu Ser Trp Ile Phe Leu Val Val Leu Phe Lys Gly
1               5                   10                  15

Val Arg Cys Glu Val Gln Ile Leu Glu Thr Gly Gly Gly Leu Val Lys
            20                  25                  30

Pro Gly Gly Ser Leu Arg Leu Ser Cys Ala Thr Ser Gly Phe Asn Phe
        35                  40                  45

Asn Asp Tyr Phe Met Asn Trp Val Arg Gln Ala Pro Glu Lys Gly Leu
    50                  55                  60

Glu Trp Val Ala Gln Ile Arg Asn Lys Ile Tyr Thr Tyr Ala Thr Phe
65                  70                  75                  80

Tyr Ala Glu Ser Leu Glu Gly Arg Val Thr Ile Ser Arg Asp Asp Ser
                85                  90                  95

Glu Ser Ser Val Tyr Leu Gln Val Ser Ser Leu Arg Ala Glu Asp Thr
            100                 105                 110

Ala Ile Tyr Tyr Cys Thr Arg Ser Leu Thr Gly Gly Asp Tyr Phe Asp
        115                 120                 125

Tyr Trp Gly Gln Gly Val Met Val Thr Val Ser Leu Ala Glu Thr Thr
    130                 135                 140

Ala Pro Ser Val Tyr Pro Leu Ala Pro Gly Thr Ala Leu Lys Ser Asn
145                 150                 155                 160

Ser Met Val Thr Leu Gly Cys
                165

<210> 22
<211> 139
<212> PRT
<213> Rattus norvegicus

<400> 22

Met Glu Thr Asp Arg Leu Leu Leu Trp Ala Leu Leu Leu Trp Val Pro
1               5                   10                  15

Gly Ser Thr Gly Asp Ile Val Leu Thr Gln Ser Pro Ala Leu Ala Val
            20                  25                  30

Ser Leu Gly Gln Arg Ala Thr Ile Ser Cys Arg Ala Ser Gln Ser Val
        35                  40                  45

Thr Ile Ser Gly Tyr Ser Phe Ile His Trp Tyr Gln Gln Lys Pro Gly
    50                  55                  60

Gln Gln Pro Arg Leu Leu Ile Tyr Arg Ala Ser Asn Leu Ala Ser Gly
65                  70                  75                  80

Ile Pro Ala Arg Phe Ser Gly Ser Gly Ser Gly Thr Asp Phe Thr Leu
                85                  90                  95

Thr Ile Asn Pro Val Gln Ala Asp Asp Ile Ala Thr Tyr Phe Cys Gln
            100                 105                 110

Gln Ser Arg Lys Ser Pro Trp Thr Phe Ala Gly Gly Thr Lys Leu Glu
        115                 120                 125

Leu Arg Arg Ala Asp Ala Ala Pro Thr Val Ser
    130                 135

## Description

### Technical Field

The present invention relates to use of an anti-Siglec-15 antibody for the treatment and/or prophylaxis of pediatric osteoporosis.

### Background Art

Osteoporosis is a disease in which bone strength is reduced due to loss of bone mass and abnormal bone substance, so that bone fracture easily occurs. Osteoporosis is developed mainly in postmenopausal women and elderly persons. However, growing children may develop osteoporosis from drugs or diseases.

The most frequent cases of onset of pediatric osteoporosis are associated with administration of drugs such as steroid drugs and immunosuppressive drugs. A large number of cases of onset have been reported in pediatric patients given the above-mentioned drugs for treating inflammatory diseases such as nephrotic syndrome. In particular, pediatric patients who have been treated with a large amount of steroid may develop significant bone fragility, resulting in bone pain or multiple spinal bone fracture. Osteoporosis caused by administration of steroid drugs is referred to as glucocorticoid-induced osteoporosis (GIO).

Other causes of pediatric osteoporosis include congenital diseases such as dysosteogenesis (designated intractable disease which is developed in one of twenty-to thirty-thousand persons). Here, bone fracture or bone deformation may repeatedly occur, resulting in delayed motor development.

When compressed fracture of the spine or fracture of limb bones frequently occurs with pediatric osteoporosis, the skeleton may be deformed, followed by persistence of disorder of movement or body trunk support functions for a lifetime. Microfracture may repeatedly occur, leading to afflicting chronic bone pain.

Currently, therapeutic agents containing a bone resorption inhibitor are administered to osteoporosis patients. The bone mineral density, bone pain and the like have been shown to be improved by administering a bisphosphonate preparation (bone resorption inhibitor) to dysosteogenesis patients. For pediatric patients, cyclic intravenous administration of pamidronate as a bisphosphonate preparation is performed, and this treatment has been covered by insurance in Japan since 2014.

However, treatment using a potent bone resorption inhibitor such as a bisphosphonate preparation is associated with a risk of developing growth disorder, renal disorder, abnormal bone substance and ureteral lithiasis with long-term oral administration. Therefore, use of such a preparation for growing children may cause development of growth disorder, abnormal bone structure/bone substance or the like. At the present time, there is not a bone resorption inhibitor which can be safely used for pediatric osteoporosis patients.

Sialic-acid-binding immunoglobulin-like lectin (hereinafter, referred to as "Siglec") is a type I membrane protein family which recognizes a sialic acid-containing sugar chain and binds thereto. It has been shown that Siglec-15 belonging to the family is preserved at a high level in the evolution of from fish to humans, and intensely expressed in dendritic cells and macrophage system cells in the human spleen and lymph node. Further, it has been shown that expression of Siglec-15 is enhanced with differentiation and maturity of osteoclasts, and when expression of Siglec-15 is reduced by RNA interference, differentiation of osteoclasts is suppressed (Patent Literature 1). Further, it has been reported that an anti-Siglec-15 antibody is capable of suppressing formation of osteoclasts and bone resorption by osteoclasts, and can be used as a drug for the treatment and/or prophylaxis of abnormal bone metabolism diseases (Patent Literature 2).

However, the action/effect of the anti-Siglec-15 antibody on pediatric osteoporosis has not been elucidated yet.

### Citation List

### Patent Literature

[Patent Literature 1] WO 2007/093042
[Patent Literature 2] WO 2009/048072

### Summary of Invention

### Technical Problem

It is an object of the present invention to provide a pharmaceutical agent capable of treating and/or preventing pediatric osteoporosis without causing growth disorder in a subject to be medicated even when the subject is a pediatric osteoporosis patient.

### Solution to Problem

The present inventors have extensively conducted studies for solving the above-described problems, and resultantly found that by administering an antibody which binds to Siglec-15, the bone mass and the bone mineral density are improved without causing bone growth disorder in a subject to be medicated, and therefore an antibody which binds to Siglec-15 is useful as a drug for the treatment and prophylaxis of osteoporosis in growing children whose bones are significantly growing. Accordingly, the present invention has been completed.

Specifically, the present invention includes the following aspects.
[1] A pharmaceutical composition for the treatment and/or prophylaxis of pediatric osteoporosis, the pharmaceutical composition comprising an antibody or a functional fragment thereof which binds to Siglec-15 and has activity of suppressing formation of osteoclasts and/or bone resorption by osteoclasts.
[2] The pharmaceutical composition of [1], wherein the antibody is a monoclonal antibody.
[3] The pharmaceutical composition of [1], wherein the antibody consists of a heavy chain containing CDRH1 consisting of the amino acid sequence set forth as SEQ ID NO: 12 in Sequence Listing, CDRH2 consisting of the amino acid sequence set forth as SEQ ID NO: 13 in Sequence Listing and CDRH3 consisting of the amino acid sequence set forth as SEQ ID NO: 14 in Sequence Listing, and a light chain containing CDRL1 consisting of the amino acid sequence set forth as SEQ ID NO: 15 in Sequence Listing, CDRL2 consisting of the amino acid sequence set forth as SEQ ID NO: 16 in Sequence Listing and CDRL3 consisting of the amino acid sequence set forth as SEQ ID NO: 17 in Sequence Listing.
[4] The pharmaceutical composition of any one of [1] to [3], wherein the antibody is a chimeric antibody, a humanized antibody or a human antibody.
[5] The pharmaceutical composition of any one of [1] to [4], wherein the functional fragment of the antibody is Fab, F(ab')₂, Fab', Fv or scFv.
[6] A method for the treatment and/or prophylaxis of pediatric osteoporosis, the method comprising administering the pharmaceutical composition of any one of [1] to [5].

The present invention also includes the following aspects.
[1] A pharmaceutical composition for the treatment and/or prophylaxis of pediatric osteoporosis, the pharmaceutical composition comprising an antibody or a functional fragment thereof which binds to Siglec-15 and has activity of suppressing formation of osteoclasts and/or bone resorption by osteoclasts.
[2] The pharmaceutical composition of [1], which does not cause growth disorder, abnormal bone structure and/or abnormal bone substance.
[3] The pharmaceutical composition of [1] or [2], wherein the pediatric osteoporosis is pediatric osteoporosis developed due to drug administration.
[4] The pharmaceutical composition of [1] or [2], wherein the pediatric osteoporosis is pediatric steroid-induced osteoporosis.
[5] The pharmaceutical composition of any one of [1] to [4], wherein the antibody is a monoclonal antibody.
[6] The pharmaceutical composition of any one of [1] to [4], wherein the antibody consists of a heavy chain containing CDRH1 consisting of the amino acid sequence set forth as SEQ ID NO: 12 in Sequence Listing, CDRH2 consisting of the amino acid sequence set forth as SEQ ID NO: 13 in Sequence Listing and CDRH3 consisting of the amino acid sequence set forth as SEQ ID NO: 14 in Sequence Listing, and a light chain containing CDRL1 consisting of the amino acid sequence set forth as SEQ ID NO: 15 in Sequence Listing, CDRL2 consisting of the amino acid sequence set forth as SEQ ID NO: 16 in Sequence Listing and CDRL3 consisting of the amino acid sequence set forth as SEQ ID NO: 17 in Sequence Listing.
[7] The pharmaceutical composition of any one of [1] to [6], wherein the antibody is a chimeric antibody, a humanized antibody or a human antibody.
[8] The pharmaceutical composition of any one of [1] to [7], wherein the functional fragment of the antibody is Fab, F(ab')₂, Fab', Fv or scFv.
[9] A method for the treatment and/or prophylaxis of pediatric osteoporosis, the method comprising administering the pharmaceutical composition of any one of [1] to [8].
[10] Use of an antibody or a functional fragment thereof which binds to Siglec-15 and has activity of suppressing formation of osteoclasts and/or bone resorption by osteoclasts, in production of a pharmaceutical composition for the treatment and/or prophylaxis of pediatric osteoporosis.
[11] An antibody or a functional fragment thereof which binds to Siglec-15 and has activity of suppressing formation of osteoclasts and/or bone resorption by osteoclasts, for use in a method for the treatment and/or prophylaxis of pediatric osteoporosis.

The matters disclosed in the description and/or the drawings of Japanese Patent Application No. 2017-129129 as a basis of priority to the present application are incorporated herein.

All the publications, patents and patent applications cited herein are incorporated herein by reference in their entirety.

### Advantageous Effect of Invention

According to the present invention, it is possible to provide a pharmaceutical agent capable of treating and/or preventing pediatric osteoporosis without causing growth disorder in a subject to be medicated even when the subject is a pediatric osteoporosis patient.

### Brief Description of Drawings

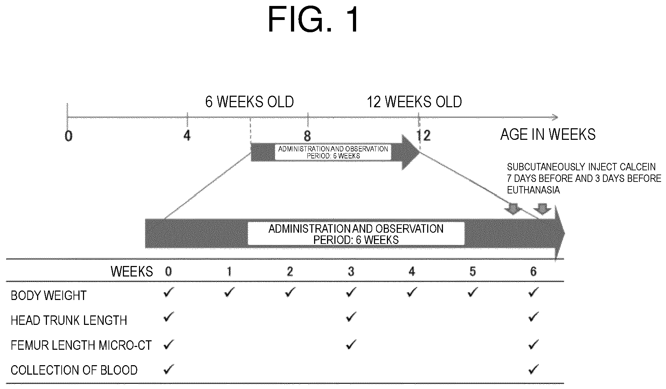

[Figure 1] Figure 1 shows schedules for carrying out various operations in experiments.

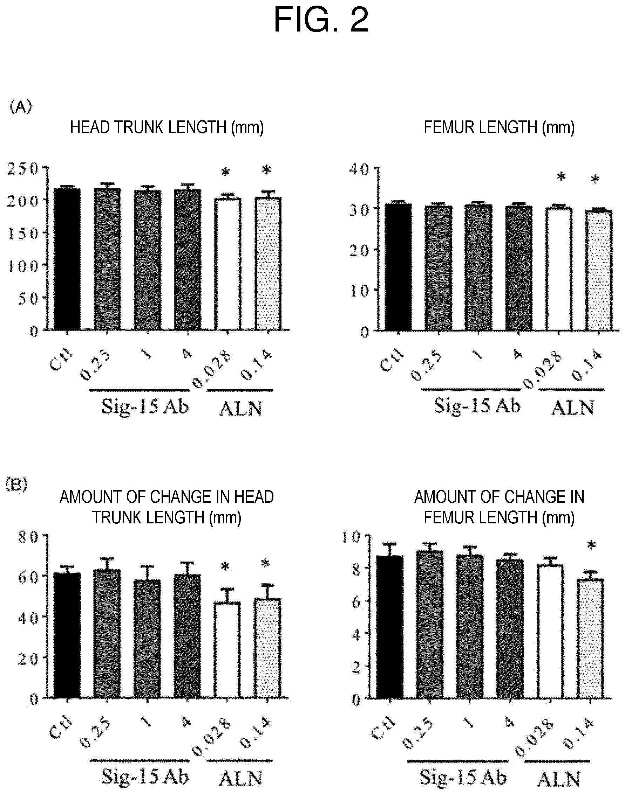

[Figure 2] Figure 2 shows graph charts showing the results of longitudinally measuring the head trunk length and the femur length over an administration and observation period for a control group (Ct1), an anti-Siglec-15 antibody administration group (Sig-15 Ab) and a bisphosphonate administration group (ALN), where Figure 2(A) shows the results of measuring the head trunk length and the femur length of each animal at the end of the administration and observation period, and Figure 2(B) shows the amounts of change in head trunk length and femur length of each animal over the administration and observation period (6 to 12 weeks in age). *: p < 0.05 (vs. Ct1).

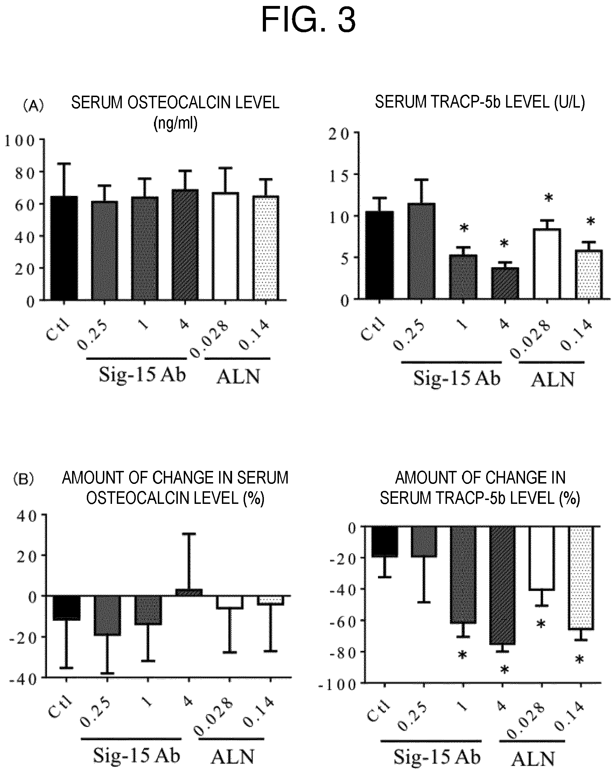

[Figure 3] Figure 3 shows graph charts showing the results of measuring a bone formation marker (serum osteocalcin) and a bone resorption marker (serum TRACP-5b) in a blood sample collected before the start of administration and 6 weeks after administration (at the age of 12 weeks) for the control group (Ct1), the anti-Siglec-15 antibody administration group (Sig-15 Ab) and the bisphosphonate administration group (ALN), where
Figure 3(A) shows the results of measuring the serum osteocalcin level and the serum TRACP-5b level of each animal at the end of the administration and observation period (at the age of 12 weeks), and Figure 3(B) shows the amounts of change in serum osteocalcin level and serum TRACP-5b level of each animal over the administration and observation period (6 to 12 weeks in age). *: p < 0.05 (vs. Ct1).

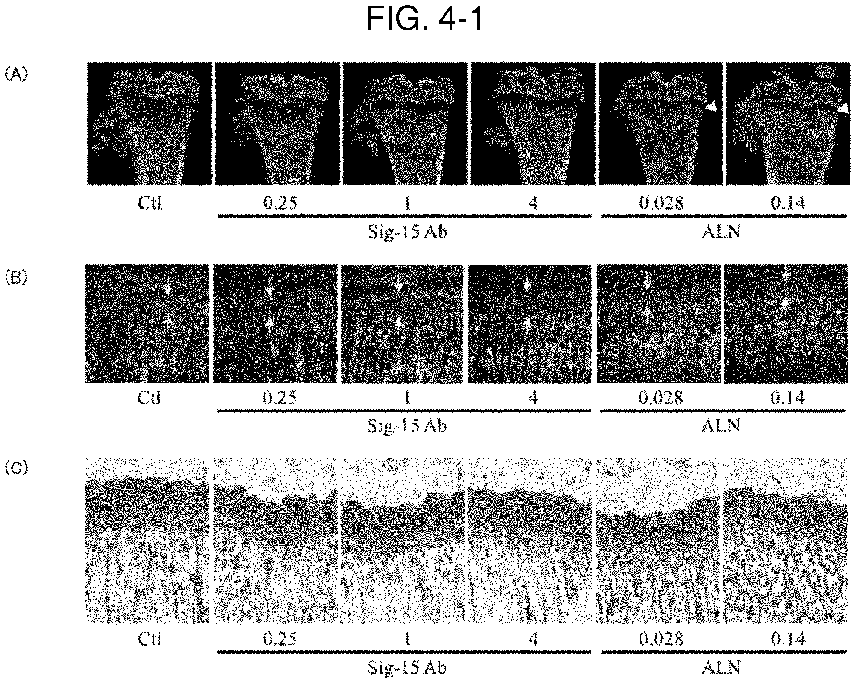

[Figure 4-1] Figure 4-1 shows the results of histologically analyzing the effect of the drug on growth for the control group (Ct1), the anti-Siglec-15 antibody administration group (Sig-15 Ab) and the bisphosphonate administration group (ALN), where Figure 4-1(A) shows coronal cross-section photographs of 3D-CT images of the proximal tibia at the age of 12 weeks, Figure 4-1(B) shows the results of Villanueva staining in a non-decalcified tissue sample prepared from a proximal tibia tissue obtained by performing labeling with calcein 7 days before and 3 days before euthanasia (the upper-side arrow indicates a region labeled 3 days before euthanasia and the lower-side arrow indicates a region labeled 7 days before euthanasia), and Figure 4-1(C) shows the results of safranine O staining (staining of acidic mucopolysaccharides) in tissue samples of the growth cartilage and a primary spongiosa region immediately below the growth cartilage at the age of 12 weeks.

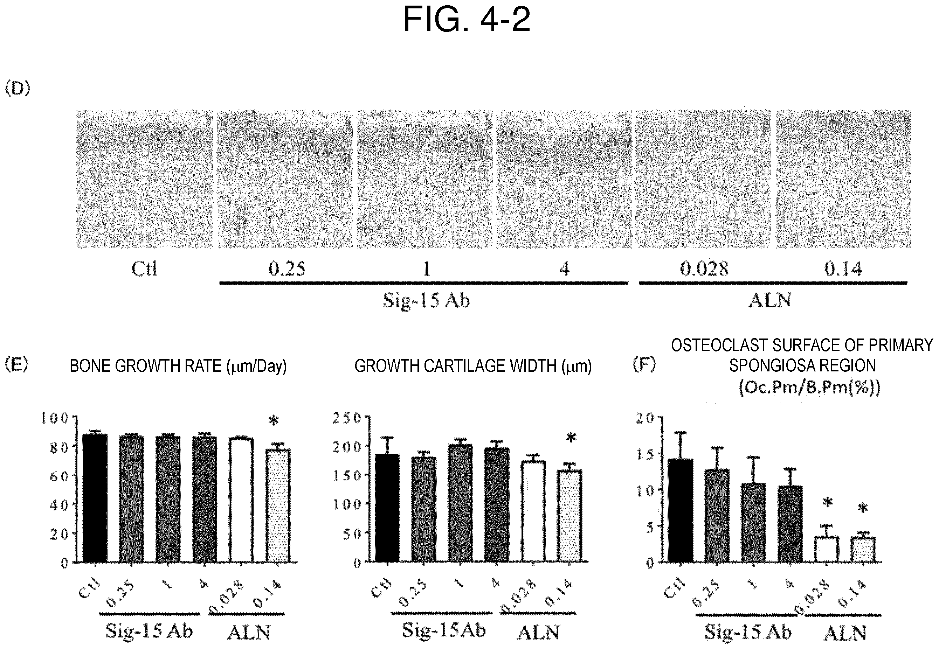

[Figure 4-2] Figure 4-2 shows the results of histologically analyzing the effect of the drug on growth for the control group (Ct1), the anti-Siglec-15 antibody administration group (Sig-15 Ab) and the bisphosphonate administration group (ALN), where Figure 4-2(D) shows the results of TRACP staining and methyl green staining in a tissue sample of a primary spongiosa region at the proximal tibia at the age of 12 weeks, Figure 4-2(E) shows bone growth rates and growth cartilage widths measured using a non-decalcified tissue sample, and
Figure 4-2(F) shows the results of measuring the ratio of the osteoclast surface to the bone surface in a primary spongiosa region (Oc.Pm/B.Pm (%)). *: p < 0.05 (vs. Ct1).

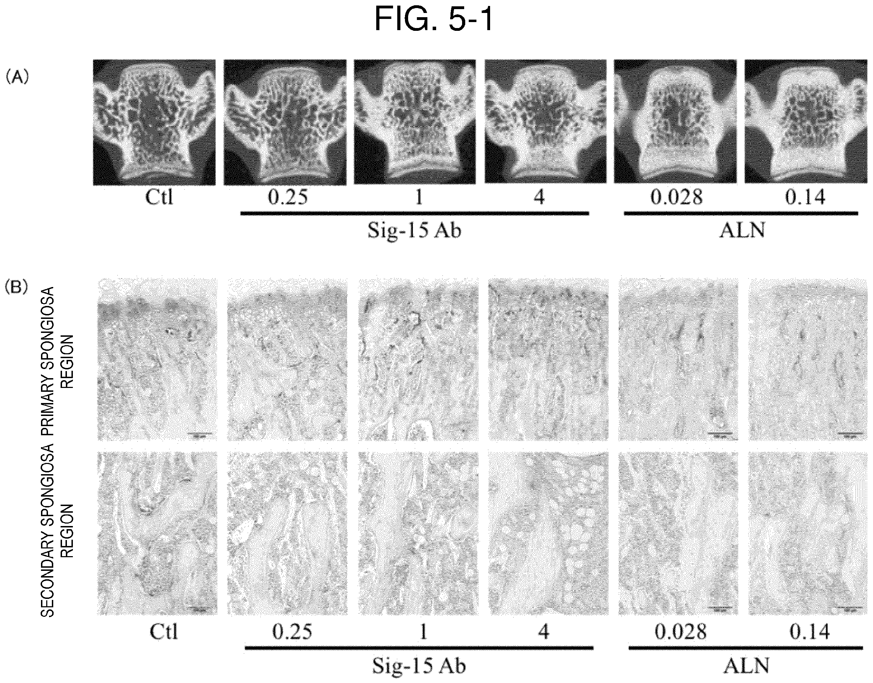

[Figure 5-1] Figure 5-1 shows the results of analyzing the effect of the drug on the bone mass and mechanical strength using the lumber vertebra for the control group (Ct1), the anti-Siglec-15 antibody administration group (Sig-15 Ab) and the bisphosphonate administration group (ALN), where Figure 5-1(A) shows coronal cross-section photographs of 3D-CT images of the fifth lumber vertebra at the age of 12 weeks, and Figure 5-1(B) shows the results of TRACP staining and methyl green staining in tissue samples of primary and secondary spongiosa regions and the vertebral body side of the fifth lumber vertebra at the age of 12 weeks.

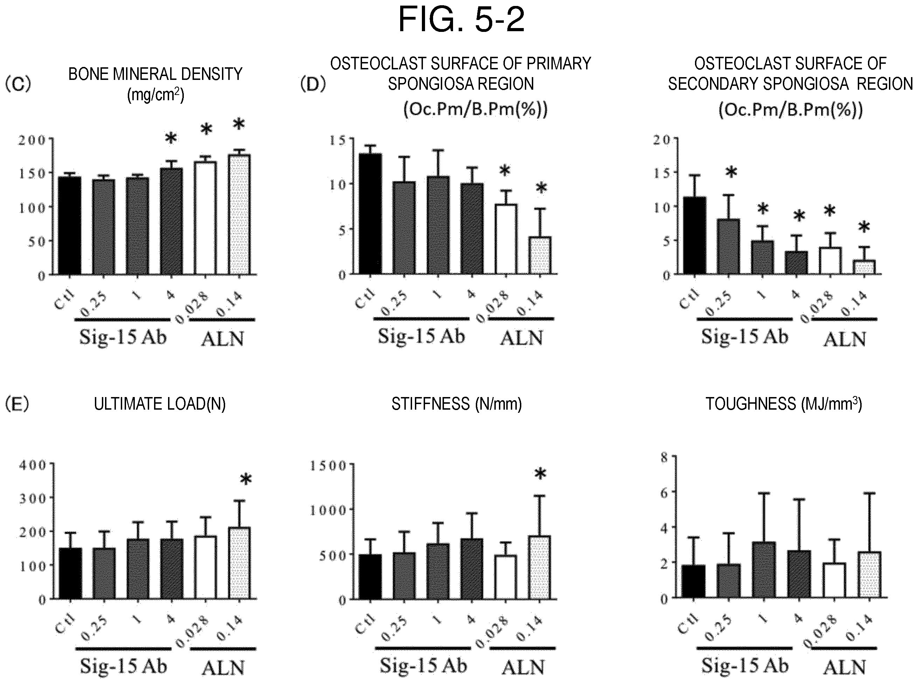

[Figure 5-2] Figure 5-2 shows the results of analyzing the effect of the drug on the bone mass and mechanical strength using the lumber vertebra for a control group (Ct1), an anti-Siglec-15 antibody administration group (Sig-15 Ab) and a bisphosphonate administration group (ALN), where Figure 5-2(C) shows the results of measuring the bone densities of the first to third lumber vertebrae at the age of 12 weeks using a DXA method, Figure 5-2(D) shows the results of measuring the ratio of the osteoclast surface to the bone surface in each of a primary spongiosa region and a secondary spongiosa region of the fifth lumber vertebra at the age of 12 weeks (Oc.Pm/B.Pm (%)), and Figure 5-2(E) shows the results of a compression mechanical test of the lumber vertebral body at the age of 12 weeks (Ultimate load, stiffness and toughness) (average value for the second, third, fourth and sixth vertebrae). *: p < 0.05 (vs. Ct1).

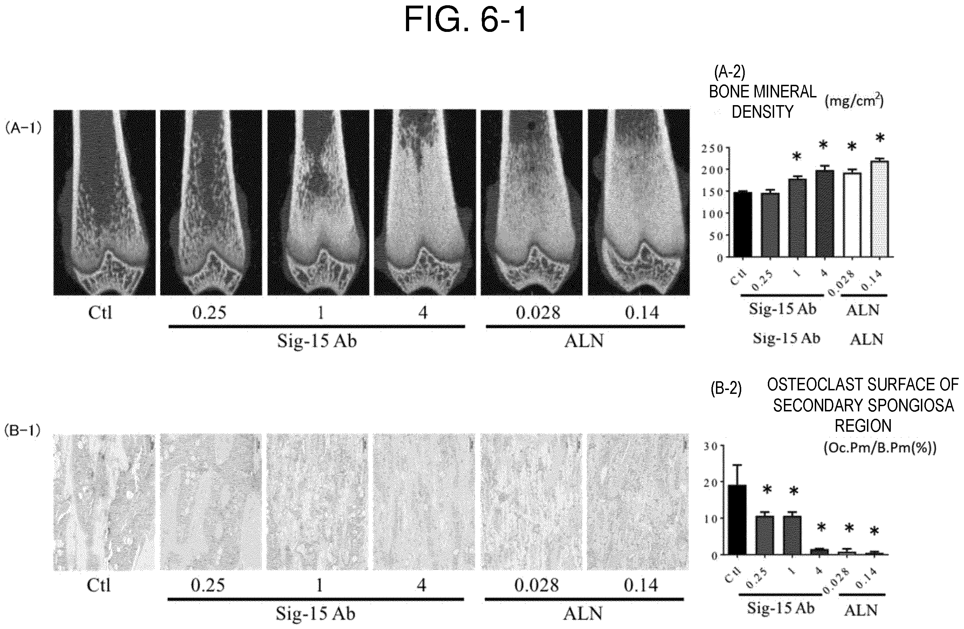

[Figure 6-1] Figure 6-1 shows the results of analyzing the effect of the drug on the bone mass and mechanical strength using the long bone for the control group (Ct1), the anti-Siglec-15 antibody administration group (Sig-15 Ab) and the bisphosphonate administration group (ALN), where Figure 6-1(A-1) shows coronal cross-section photographs of 3D-CT images of the distal femur at the age of 12 weeks, Figure 6-1(A-2) shows the results of measuring the bone mineral density of the distal femur at the age of 12 weeks using a DXA method, Figure 6-1(B-1) shows the results of TRACP staining and methyl green staining in a tissue sample of a secondary spongiosa region of the proximal tibia at the time of 12 weeks in age, and Figure 6-1(B-2) shows the results of measuring the ratio of the osteoclast surface to the bone surface in the proximal tibia at the time of 12 weeks in age (Oc.Pm/B.Pm (%)).

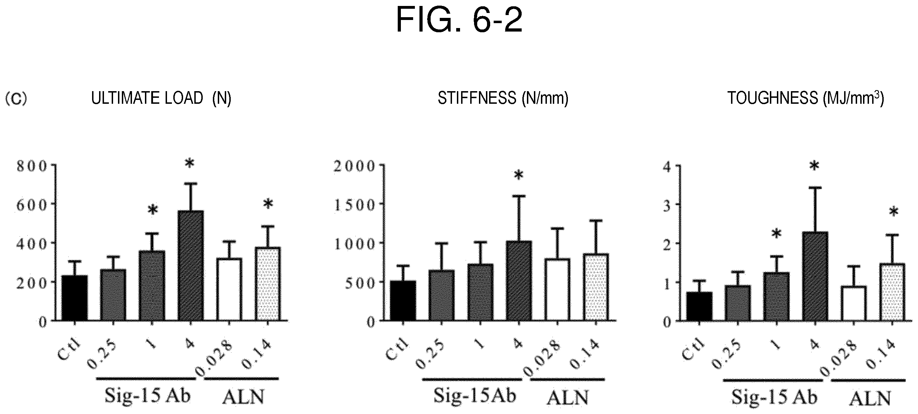

[Figure 6-2] Figure 6-2 shows the results of analyzing the effect of the drug on the bone mass and mechanical strength using the long bone for the control group (Ct1), the anti-Siglec-15 antibody administration group (Sig-15 Ab) and the bisphosphonate administration group (ALN), where Figure 6-2(C) shows the results of a compression mechanical test of the distal femur metaphysis at the age of 12 weeks (ultimate load, stiffness and toughness) (average value for the second, third, fourth and sixth vertebrae). *: p < 0.05 (vs. Ct1).

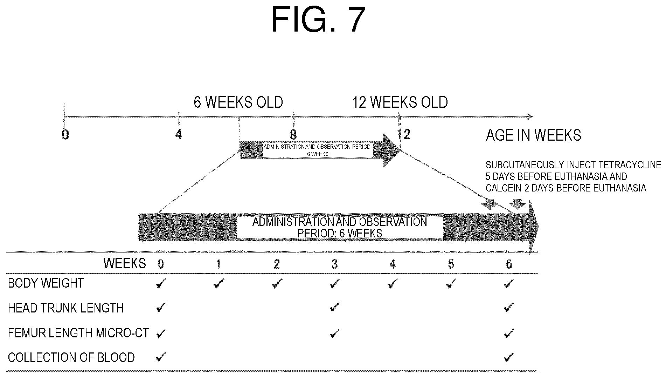

[Figure 7] Figure 7 shows schedules for carrying out various operations in experiments.

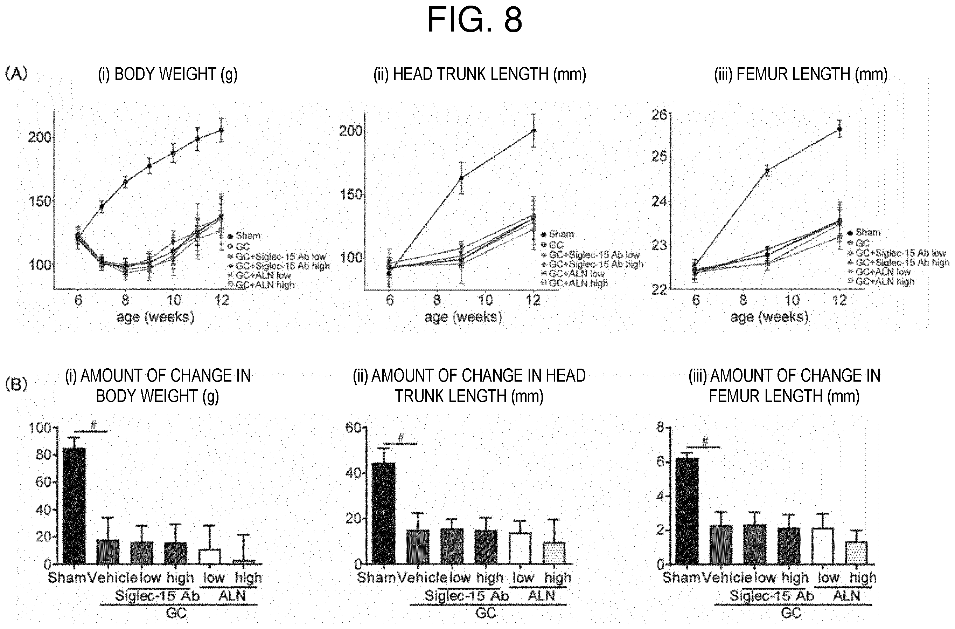

[Figure 8] Figure 8 shows graph charts showing the results of longitudinally measuring the body weight, the head trunk length and the femur length over an administration and observation period for a Sham group, a GC group (Vehicle), a GC+Siglec-15Ab group (given an anti-Siglec-15 antibody at a low dose or a high dose) and a GC+ALN group (given ALN at a low dose or a high dose), where Figure 8(A) shows the results of measuring (i) weight body, (ii) head trunk length and (iii) femur length of each animal over the administration and observation period and at the end of the period, and
Figure 8(B) shows the amounts of change in (i) body weight, (ii) head trunk length and (iii) femur length of each animal over the administration and observation period (6 to 12 weeks in age). #; p < 0.05 (vs. sham group).

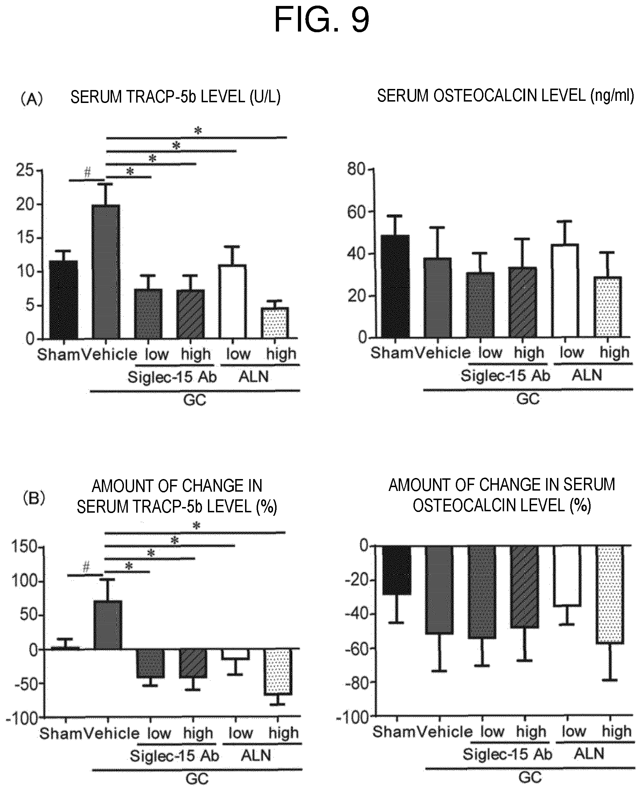

[Figure 9] Figure 9 shows graph charts showing the results of measuring a bone resorption marker (serum TRACP-5b) and a bone formation marker (serum osteocalcin) in a blood sample collected before the start of administration and 6 weeks after administration (at the age of 12 weeks) for the Sham group, the GC group (Vehicle), the GC+Siglec-15Ab group (given an anti-Siglec-15 antibody at a low dose or a high dose) and the GC+ALN group (given ALN at a low dose or a high dose), where Figure 9(A) shows the results of measuring the serum TRACP-5b level and the serum osteocalcin level of each animal at the end of the administration and observation period (at the age of 12 weeks), and Figure 9(B) shows the amounts of change in serum TRACP-5b level and serum osteocalcin level of each animal over the administration and observation period (6 to 12 weeks in age) as a ratio of change from the level at the start of administration (6 weeks in age). #; p < 0.05 (vs. Sham group), *; p < 0.05 (vs. GC group).

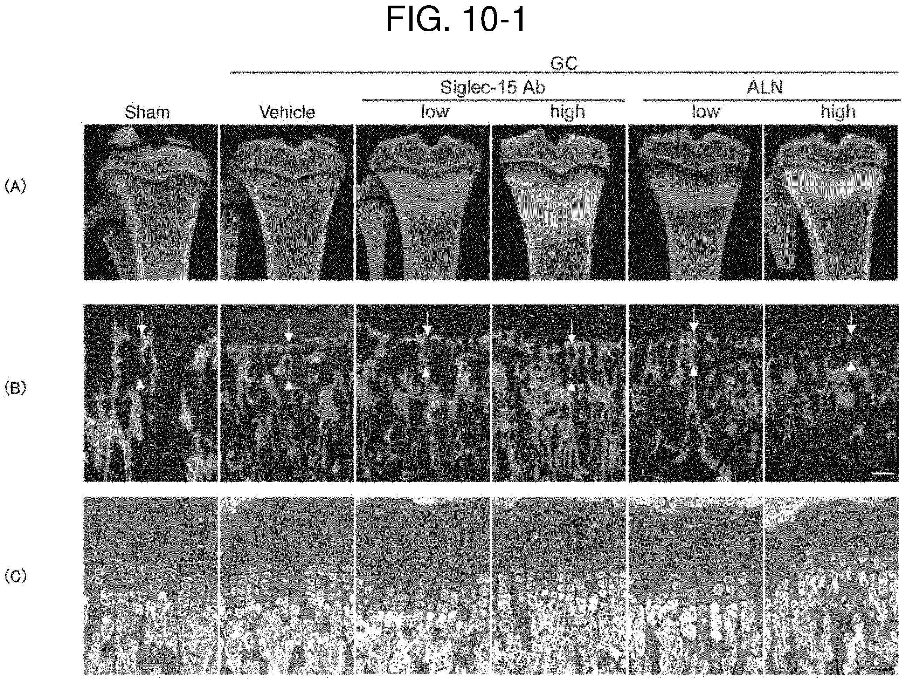

[Figure 10-1] Figure 10-1 shows the results of histologically analyzing the effect of the drug on growth for the Sham group, the GC group (Vehicle), the GC+Siglec-15Ab group (given an anti-Siglec-15 antibody at a low dose or a high dose) and the GC+ALN group (given ALN at a low dose or a high dose), where Figure 10-1(A) shows coronal cross-section photographs of 3D-CT images of the proximal tibia at the age of 12 weeks, Figure 10-1(B) shows the results of Villanueva staining in a non-decalcified tissue sample prepared from a proximal tibia tissue obtained by performing labeling with tetracycline 5 days before euthanasia and with calcein 2 days before euthanasia (the upper-side arrow indicates a region labeled 2 days before euthanasia and the lower-side arrow indicates a region labeled 5 days before euthanasia), and
Figure 10-1(C) shows the results of safranine O staining (staining of acidic mucopolysaccharides) in tissue samples of the growth cartilage and a primary spongiosa region immediately below the growth cartilage at the age of 12 weeks.

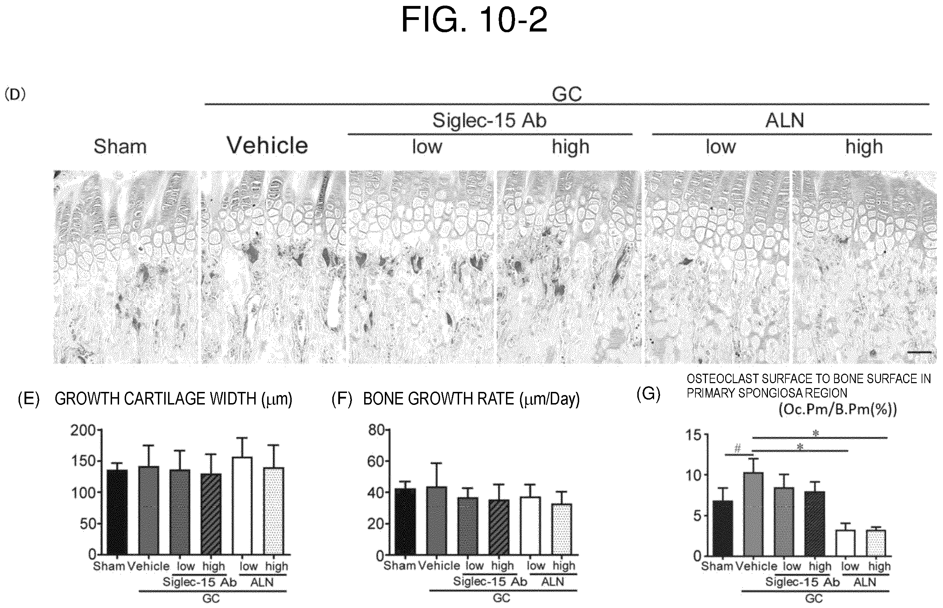

[Figure 10-2] Figure 10-2 shows the results of histologically analyzing the effect of the drug on growth for the Sham group, the GC group (Vehicle), the GC+Siglec-15Ab group (given an anti-Siglec-15 antibody at a low dose or a high dose) and the GC+ALN group (given ALN at a low dose or a high dose), where Figure 10-2(D) shows the results of TRACP staining and methyl green staining in a tissue sample of a primary spongiosa region at the proximal tibia at the age of 12 weeks, and Figures 10-2(E), 10-2(F) and 10-2(G) show the results of
   measuring the growth cartilage width, the bone growth rate, and the ratio of the osteoclast surface to the bone surface in a primary spongiosa region (Oc.Pm/B.Pm (%)), respectively, using a non-decalcified tissue sample. #; p < 0.05 (vs. Sham group), *; p <0.05 (vs. GC group).

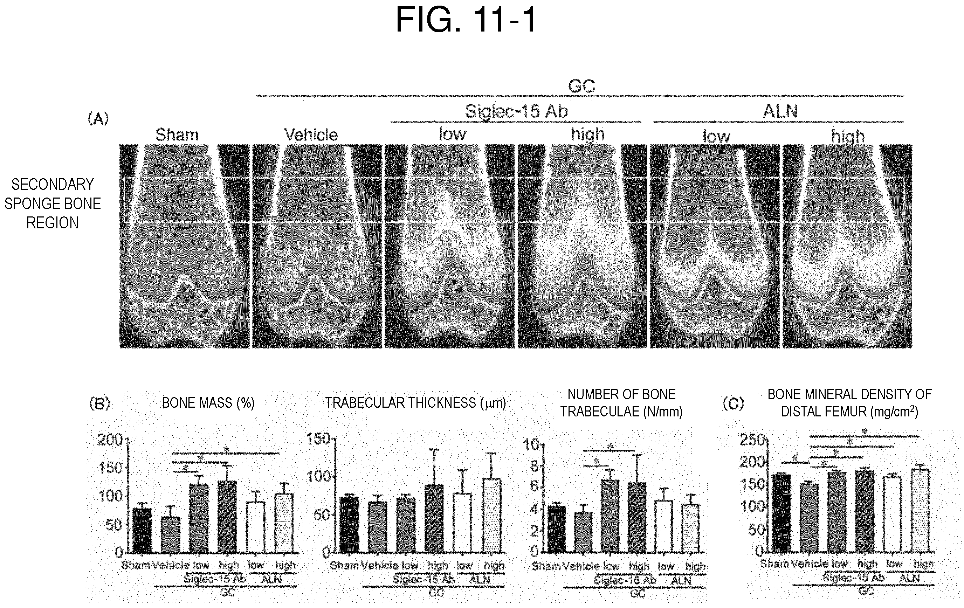

[Figure 11-1] Figure 11-1 shows the results of analyzing the effect of the drug on the bone mass and mechanical strength using the long bone for the Sham group, the GC group (Vehicle), the GC+Siglec-15Ab group (given an anti-Siglec-15 antibody at a low dose or a high dose) and the GC+ALN group (given ALN at a low dose or a high dose), where Figure 11-1(A) shows coronal cross-section photographs of 3D-CT images of the distal femur at the age of 12 weeks (the regions surrounded by a rectangle represent secondary spongiosa regions), Figure 11-1(B) shows the results of measuring the bone mass BV/TV (%), the bone trabecula thickness Tb.Th (µm) and the number of bone trabeculae Tb.N (N/mm) of the secondary spongiosa region at the age of 12 weeks, and Figure 11-1(C) shows the results of measuring the bone mineral density BMD of the distal femur using a DXA method. #; p < 0.05 (vs. Sham group), *; p <0.05 (vs. GC group).

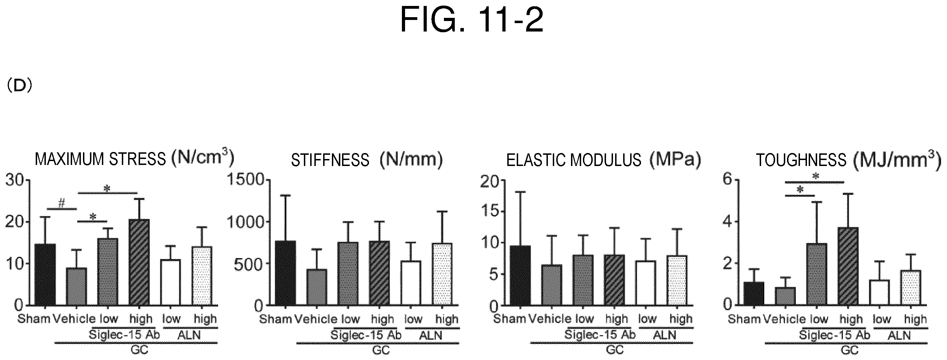

[Figure 11-2] Figure 11-2(D) shows the results of a compression mechanical test of the distal femur metaphysis at the age of 12 weeks (maximum stress, stiffness, elastic modulus and toughness) for the Sham group, the GC group (Vehicle), the GC+Siglec-15Ab group (given an anti-Siglec-15 antibody at a low dose or a high dose) and the GC+ALN group (given ALN at a low dose or a high dose). #; p < 0.05 (vs. Sham group), *; p <0.05 (vs. GC group).

### Description of Embodiments

Herein, the term "gene" includes not only DNA but also mRNA, cDNA and cRNA.

Herein, the term "polynucleotide" is used for the same meaning as nucleic acid, and includes DNA, RNA, probes, oligonucleotides and primers.

Herein, the term "polypeptide" and the term "protein" are used without distinction.

Herein, the term "cell" includes cells in individual animals, and cultured cells.

Herein, the term "Siglec-15" is used for the same meaning as "Siglec-15 protein".

Herein, the term "formation of osteoclasts" is used for the same meaning as "differentiation of osteoclasts" or "maturity of osteoclasts".

The "functional fragment of the antibody" herein means a sub-fragment of an antibody having binding activity with an antigen, and includes Fab, F(ab')₂ and scFv. The functional fragments of the antibody also include Fab' which is a univalent fragment of a variable region of an antibody obtained by treating F(ab')₂ under reducing conditions. However, the fragment is not limited to these molecules as long as it has a binding ability with an antigen. These functional fragments include not only antibody proteins with overall molecules treated with appropriate enzymes, but also proteins produced in appropriate host cells using antibody genes modified by genetic engineering.

The "epitope" herein means a sub-peptide of Siglec-15 to which a specific anti-Siglec-15 antibody binds. The epitope which is the sub-peptide of Siglec-15 can be determined by a method well known to those skilled in the art, such as an immunological assay method. Examples thereof include the following method. Various sub-structures of Siglec-15 are prepared. In preparation of the sub-structures, a known oligopeptide synthesis technique can be used. For example, a series of polypeptides having an appropriate length from the C-terminal or the N-terminal of Siglec-15 and descending in length sequentially are prepared using a genetic recombination technique known to those skilled in the art, reactivity of the antibody to these polypeptides is then examined to roughly determine a recognition site, shorter peptides are then synthesized, and reactivity with the peptides is examined, whereby the epitope can be determined. When a second anti-Siglec-15 antibody binds to a sub-peptide to which a first anti-Siglec-15 antibody binds, it can be determined that the first antibody and the second antibody have a common epitope. Further, by confirming that the second anti-Siglec-15 antibody competes against the first anti-Siglec-15 antibody binding to Siglec-15 (i.e. the second antibody hinders binding between the first antibody and Siglec-15), it can be determined that the first antibody and the second antibody have a common epitope even when a specific epitope sequence is not determined. Further, when the first antibody and the second antibody bind to a common epitope, and the first antibody has a particular effect such as antigen-neutralizing activity, the second antibody is expected to have similar activity.

In the present invention, the phrase "hybridizing under stringent conditions" means hybridizing at 68°C in a commercially available hybridization solution "ExpressHyb Hybridization Solution" (TAKARA BIO INC.), or hybridizing under conditions enabling identification to be performed by carrying out hybridization at 68°C in the presence of 0.7 to 1.0 M NaCl with a filter on which DNA is immobilized, and then performing washing at 68°C using a SSC solution with a 0.1- to 2-fold concentration (SSC with a 1-fold concentration is composed of 150 mM NaCl and 15 mM sodium citrate), or equivalent conditions.

### 1. Siglec-15

The Siglec-15 gene is a gene confirmed to be expressed at a significantly higher level in a giant cell tumor (GCT). Further, the Siglec-15 gene is a gene confirmed to be expressed at a higher level in differentiation of monocytic cell-derived cell lines into osteoclasts (WO 2009/048072).

Siglec-15 for use in the present invention can be obtained by purifying Siglec-15 directly from monocytic cells or bone-marrow cells of a human, a non-human mammal (e.g. guinea pig, rat, mouse, rabbit, pig, sheep, bovine or monkey) or a chicken, preparing membrane fractions of the cells, synthesizing Siglec-15 *in vitro,* or causing host cells to produce Siglec-15 through genetic manipulation. In genetic manipulation, specifically, cDNA of Siglec-15 is incorporated into a vector capable of expressing the cDNA, and Siglec-15 is synthesized in a solution containing an enzyme, a substrate and an energetic substance which are required for transcription and translation, or host cells of another prokaryotic organism or eukaryotic organism are transformed to express Siglec-15, whereby the protein can be obtained.

The nucleotide sequence of cDNA of human Siglec-15 is registered as accession number: NM_213602 in GenBank, and set forth as SEQ ID NO: 1 in Sequence Listing, and the amino acid sequence thereof is set forth as SEQ ID NO: 2 in Sequence Listing. The nucleotide sequence of cDNA of mouse Siglec-15 is registered as accession number: XM_884636 in GenBank, and set forth as SEQ ID NO: 3 in Sequence Listing, and the amino acid sequence thereof is set forth as SEQ ID NO: 4 in Sequence Listing. Mature human Siglec-15 from which the signal sequence has been removed corresponds to an amino acid sequence consisting of amino acid residues at positions 21 to 328 in the amino acid sequence set forth as SEQ ID NO: 2. Mouse Siglec-15 from which the signal sequence has been removed corresponds to an amino acid sequence consisting of amino acid residues at positions 21 to 341 in the amino acid sequence set forth as SEQ ID NO: 4. Siglec-15 is sometimes called CD33 antigen-like 3, CD33 molecule-like 3, CD33-like 3 or CD33L3, and they denote the same molecule.

cDNA of Siglec-15 can be acquired by, for example, a so-called PCR method in which a cDNA library expressing cDNA of Siglec-15 is provided as a template, and polymerase chain reaction (hereinafter, referred to as "PCR") is carried out using a primer that specifically amplifies cDNA of Siglec-15 (Saiki, R.K., et al., Science, (1988)239, 487-49).

cDNA of Siglec-15 includes a polynucleotide which hybridizes under stringent conditions with a polynucleotide consisting of nucleotide sequence(s) complementary to nucleotide sequence(s) set forth as at least one selected from SEQ ID NOS: 1 and 3 in Sequence Listing and which encodes a protein equivalent in biological activity to Siglec-15. Further, cDNA of Siglec-15 includes a splicing variant which is transcribed from a human or mouse Siglec-15 gene locus, or a polynucleotide which hybridizes under stringent conditions with the splicing variant and which encodes a protein equivalent in biological activity to Siglec-15.

Further, Siglec-15 includes a protein consisting of amino acid sequence(s) set forth as at least one selected from SEQ ID NOS: 2 and 4 in Sequence Listing, or the amino acid sequence(s) from which the signal sequence has been removed and in which one or several amino acids are substituted, deleted or added, the protein being equivalent in biological activity to Siglec-15. Further, Siglec-15 includes a protein consisting of an amino acid sequence which is encoded by a splicing variant transcribed from a human or Siglec-15 gene locus, or the amino acid sequence in which one or several amino acids are substituted, deleted or added, the protein being equivalent in biological activity to Siglec-15.

### 2. Production of anti-Siglec-15 antibody

Using a conventional method, the antibody to Siglec-15 according to the present invention can be obtained by immunizing an animal with Siglec-15 or any polypeptide selected from the amino acid sequences of Siglec-15, and collecting and purifying an antibody produced in the organism. Species for Siglec-15 as an antigen are not limited to humans, and the animal can be immunized with Siglec-15 derived from non-human animals such as mice and rats. In this case, by examining cross-reactivity between an antibody binding to acquired heterologous Siglec-15 and human Siglec-15, an antibody applicable to a human disease can be selected.

In accordance with a known method (e.g. Kohler and Milstein, Nature (1975) 256, p.495-497, Kennet, R. ed., Monoclonal Antibody, p.365-367, Prenum Press, N.Y. (1980)), antibody producing cells which produce an antibody to Siglec-15 are fused with myeloma cells, whereby hybridoma can be established to obtain a monoclonal antibody.

Siglec-15 as an antigen can be obtained by causing host cells to produce a Siglec-15 gene through genetic manipulation.

Specifically, a vector capable of expressing a Siglec-15 gene is prepared, and introduced into host cells to express the gene, and the expressed Siglec-15 is purified. A method for acquiring an antibody to Siglec-15 will be described in detail below. Hereinafter, unless otherwise specified, operations related to genetic manipulation can be carried out in accordance with the method described in "Molecular Cloning, Vol. 4" (written by Sambrook, J., Fritsch, E.F. and Maniatis, T., published by Cold Spring Harbor Laboratory Press in 2012) .

### (1) Preparation of antigen

Examples of the antigen for preparing an anti-Siglec-15 antibody include Siglec-15, polypeptides consisting of at least 6 consecutive sub-amino acid sequences of the Siglec-15, and derivatives with any amino acid sequence or carrier added to Siglec-15 or the polypeptides. The antigen can be selected from, for example, polypeptides consisting of the amino acid sequences shown in (a) to (i) below:
(a) an amino acid sequence set forth as SEQ ID NO: 2 in Sequence Listing;
(b) an amino acid sequence consisting of amino acid residues at positions 21 to 328 in the amino acid sequence set forth as SEQ ID NO: 2 in Sequence Listing;
(c) an amino acid sequence consisting of amino acid residues at positions 1 to 260 in the amino acid sequence set forth as SEQ ID NO: 2 in Sequence Listing;
(d) an amino acid sequence consisting of amino acid residues at positions 21 to 260 in the amino acid sequence set forth as SEQ ID NO: 2 in Sequence Listing;
(e) an amino acid sequence set forth as SEQ ID NO: 4 in Sequence Listing;
(f) an amino acid sequence consisting of amino acid residues at positions 21 to 341 in the amino acid sequence set forth as SEQ ID NO: 4 in Sequence Listing;
(g) an amino acid sequence consisting of amino acid residues at positions 1 to 258 in the amino acid sequence set forth as SEQ ID NO: 4 in Sequence Listing;
(h) an amino acid sequence consisting of amino acid residues at positions 21 to 258 in the amino acid sequence set forth as SEQ ID NO: 4 in Sequence Listing; and
(i) an amino acid sequence derived from the amino acid sequences (a) to (h) by substitution, deletion or addition of one to several amino acid residues.

As the antigen, polypeptides consisting of amino acid sequences which are encoded by the nucleotide sequences shown in (j) to (n) below can be used:
(j) a nucleotide sequence set forth as SEQ ID NO: 1;
(k) a nucleotide sequence set forth as SEQ ID NO: 3;
(1) a nucleotide sequence set forth as SEQ ID NO: 5;
(m) a nucleotide sequence set forth as SEQ ID NO: 6; and
(n) a nucleotide sequence of a polynucleotide which hybridizes under stringent conditions with polynucleotides consisting of nucleotide sequences complimentary to the nucleotide sequences of (j) to (m).

The polypeptide consisting of amino acid residues at positions 1 to 20 in the amino acid sequence set forth as SEQ ID NO: 2 in Sequence Listing corresponds to the signal peptide of human Siglec-15, and the polypeptide consisting of amino acid residues at positions 21 to 260 corresponds to the extracellular region of a mature protein of human Siglec-15. The polypeptide consisting of amino acid residues at positions 1 to 20 in the amino acid sequence set forth as SEQ ID NO: 4 in Sequence Listing corresponds to the signal peptide of mouse Siglec-15, and the polypeptide consisting of amino acid residues at positions 21 to 258 corresponds to the extracellular region of a mature protein of mouse Siglec-15. Further, the nucleotide sequence set forth as SEQ ID NO: 6 encodes the extracellular region of human Siglec-15 which is encoded by the nucleotide sequence set forth as SEQ ID NO: 1, and the nucleotide sequence set forth as SEQ ID NO: 5 encodes the extracellular region of mouse Siglec-15 which is encoded by the nucleotide sequence set forth as SEQ ID NO: 3.

Siglec-15 can be purified directly from human tumor tissues or tumor cells, or obtained by synthesizing Siglec-15 *in vitro,* or causing host cells to produce Siglec-15 through genetic manipulation.

In genetic manipulation, specifically, cDNA of Siglec-15 is incorporated into a vector capable of expressing the cDNA, and Siglec-15 is synthesized in a solution containing an enzyme, a substrate and an energetic substance which are required for transcription and translation, or host cells of another prokaryotic organism or eukaryotic organism are transformed to express Siglec-15, whereby the antigen can be obtained.

It is also possible to obtain the antigen as a secreted protein by expressing in an appropriate host/vector system a fused protein in which the extracellular region of Siglec-15 that is a membrane protein is connected to the constant region of an antibody.

cDNA of Siglec-15 can be acquired by, for example, a so-called PCR method in which a cDNA library expressing cDNA of Siglec-15 is provided as a template, and polymerase chain reaction (hereinafter, referred to as "PCR") is carried out using a primer that specifically amplifies cDNA of Siglec-15 (Saiki, R.K., et al., Science, (1988)239, p.487-489).

Examples of the system for *in vitro* synthesis of polypeptides include, but are not limited to, Rapid Translation System (RTS) manufactured by Roche Diagnostics K.K.

Examples of the host of prokaryotic cells include Escherichia coli and Bacillus subtilis. For transforming a target gene in such host cells, host cells are transformed with a replicon, i.e. a replication origin, derived from a species compatible with the host, and a plasmid vector containing a regulatory sequence. The vector is preferably one having a sequence capable of imparting phenotypic character (phenotype) selectivity to the transformed cells.

The host cells of eukaryotic cells include cells of vertebrate animals, insects, yeasts and so on, and examples of the vertebrate animal cells that are commonly used include, but are not limited to, COS cells which are cells of monkeys (Gluzman, Y. Cell (1981) 23, p.175-182, ATCC CRL-1650), mouse fibroblastic cells NIH3T3 (ATCC No. CRL-1658), and dihydrofolate reductase-deficient lines (Urlaub, G. and Chasin, L. A. Proc. Natl. Acad. Sci. USA (1980) 77, p.4126-4220) of Chinese hamster ovary cells (CHO cells, ATCC CCL-61).

Transformants obtained in the manner described above can be cultured in accordance with a conventional method, and through the culture, a desired polypeptide is intracellularly or extracellularly produced.

The medium to be used for the culture can be appropriately selected from common media according to employed host cells. When the host is Escherichia coli, for example, it is possible to use a LB medium to which an antibiotic substance such as ampicillin or IPMG is added if necessary.

A recombinant protein intracellularly or extracellularly produced in the transformant by the culture can be separated and purified by various known separation operation methods using the physical and chemical properties of the protein.

Specific examples of the method include treatments with a normal protein precipitating agent, ultrafiltration, various kinds of liquid chromatography such as molecular sieve chromatography (gel filtration), adsorption chromatography, ion-exchange chromatography and affinity chromatography, dialysis, and combinations thereof.

By connecting histidine consisting of 6 residues to a recombinant protein to be expressed, the recombinant protein can be efficiently purified with a nickel affinity column. Alternatively, by connecting the Fc region of IgG to a recombinant protein to be expressed, the recombinant protein can be efficiently purified with a protein A column. By combining the above-described methods, a large amount of a desired polypeptide can be easily produced with a high yield and high purity.

### (2) Production of anti-Siglec-15 monoclonal antibody

Examples of the antibody which binds specifically to Siglec-15 include monoclonal antibodies which bind specifically to Siglec-15, and a method for acquiring the monoclonal antibodies is as described below.

In production of a monoclonal antibody, it is generally necessary to carry out the following operation steps:
(a) purification of biological polymer used as an antigen;
(b) step of injecting an antigen into an animal to immunize the animal, then collecting blood, and evaluating the antibody value thereof to determine the time of isolation of the spleen, followed by preparing antibody producing cells;
(c) preparation of myeloma cells (hereinafter, referred to as "myeloma");
(d) cellular fusion of antibody producing cells and myeloma;
(e) selection of a hybridoma group which produces a desired antibody;
(f) division into single-cell clones (cloning);
(g) optional culture of hybridoma for producing a large amount of a monoclonal antibody, or breeding of an animal implanted with hybridoma;
(h) examination of the physiological activity and the binding specificity of the monoclonal antibody thus produced, or evaluation of properties as a labeling reagent; etc.

A method for preparing a monoclonal antibody will be described in detail below in line with the above-described steps, and the method for preparing the antibody is not limited to these steps. For example, antibody producing cells other than spleen cells and myeloma can be used.

### (a) Purification of antigen

As the antigen, Siglec-15 prepared by the above-described method, or part thereof can be used.

Membrane fractions prepared from Siglec-15 expression recombinant cells, Siglec-15-expressing recombinant cells themselves, or a sub-peptide of the protein according to the present invention, which is chemically synthesized using a method known to those skilled in the art, can also be used as the antigen.

### (b) Preparation of antibody producing cells

The antigen obtained in step (a) is mixed with a complete or incomplete Freund's adjuvant or an auxiliary agent such as potash alum, and an experimental animal is immunized with the resulting mixture as an immunogen. For the experimental animal, an animal which is used for a known method for preparing hybridoma can be used without difficulty. Specifically, for example, a mouse, a rat, a goat, sheep, a bovine, a horse or the like can be used. From the viewpoint of availability of myeloma cells to be fused with isolated antibody producing cells, it is preferable to use a mouse or a rat as an animal to be immunized.

The strains of mice and rats that are actually used are not particularly limited. For mice, for example, the strains A, AKR, BALB/c, BDP, BA, CE, C3H, 57BL, C57BL, C57L, DBA, FL, HTH, HT1, LP, NZB, NZW, RF, R, III, SJL, SWR, WB, 129 and the like can be used. For rats, for example, Wistar, Low, Lewis, Spraque, Daweley, ACI, BN, Fischer and the like can be used.

These mice and rats can be acquired from experimental animal breeding and distributing companies such as CLEA Japan, Inc. and CHARLES RIVER LABORATORIES JAPAN, INC.

Among them, the BALB/c strain in mice and the Wistar and Low strains in rats are particularly preferable as animals to be immunized, in light of fusion compatibility with myeloma cells as described later.

Further, considering antigenic homology between a human and a mouse, it is preferable to use mice having a reduced biological mechanism for removing the autoantibody, i.e. autoimmune disease mice.

At the time of immunization, the mice or rats are preferably 5- to 12-week old, more preferably 6- to 8-week old.

For immunizing the animal with Siglec-15 or a recombinant thereof, a known method as described in detail in, for example, Weir, D.M., Handbook of Experimental Immunology Vol. I. II. III., Blackwell Scientific Publications, Oxford (1987), Kabat, E.A. and Mayer, M.M., Experimental Immunochemistry, Charles C Thomas Publisher Spigfield, Illinois (1964), etc. can be used.

A specific example of a method preferred in the present invention, among the immunization methods, is as follows.

First, membrane protein fractions as an antigen, or cells caused to express an antigen are intracutaneously or intraperitoneally administered to the animal.

For enhancing immunological efficiency, it is preferable to perform both the types of administration, and when intracutaneous administration is performed in the earlier half, and intraperitoneal administration is performed in the latter half or only in the final installment, immunological efficiency can be particularly enhanced.

The antigen administration schedule varies depending on the type, the interindividual difference or the like of the animal to be immunized, and in general, the antigen is administered preferably at an antigen administration frequency of 3 to 6 times and administration intervals of 2 to 6 weeks, more preferably at an antigen administration frequency of 3 or 4 times and administration intervals of 2 to 4 weeks.

The dosage of the antigen varies depending on the type, the interindividual difference or the like of the animal, and is generally about 0.05 to 5 mg, preferably about 0.1 to 0.5 mg.

Additional immunization is performed 1 to 6 weeks after, preferably 2 to 4 weeks after, more preferably 2 to 3 weeks after antigen administration as above.

The antigen dosage at the time of performing additional immunization varies depending on the type, the size or the like of the animal, and is generally, for example, about 0.05 to 5 mg, preferably about 0.1 to 0.5 mg, more preferably about 0.1 to 0.2 mg for mice.

1 to 10 days, preferably 2 to 5 days, more preferably 2 or 3 days after the additional immunization, spleen cells or lymphocytes including antibody producing cells are aseptically extracted from the immunized animal.

When the antibody value is measured here, and an animal having a sufficiently high antibody value is used as a source of antibody producing cells, the efficiency of subsequent operations can be enhanced.

Examples of the method for measuring the antibody value used here include, but are not limited to, a RIA method and an ELISA method.

For example, by the ELISA method, measurement of the antibody value in the present invention can be performed in accordance with the procedure described below.

First, a purified or partially purified antigen is adsorbed to a solid surface of a 96-well plate for ELISA or the like, a solid surface to which the antigen is not adsorbed is covered with a protein unrelated to the antigen, e.g. bovine serum albumin (hereinafter, referred to as "BSA"), and the solid surface is washed, and then brought into contact with a serially diluted sample (e.g. mouse serum) as a first antibody to bind the antibody in the sample to the antigen.

Further, as a second antibody, an enzyme-labeled antibody to a mouse antibody is added, and bound to the mouse antibody, washing is performed, a substrate of the enzyme is then added, and a change in absorbance due to color development by decomposition of the substrate, or the like is measured to calculate the antibody value.

Separation of antibody producing cells from the spleen cells or lymphocytes can be performed in accordance with a known method (e.g. Kohler et al., Nature (1975) 256, p.495; Kohler et al., Eur. J. Immnol. (1977) 6, p.511; Milstein et al., Nature (1977), 266, p.550; Walsh, Nature (1977) 266, p.495).

For example, in the case of spleen cells, a general method can be employed in which the spleen is cut into small pieces, and the cells are filtered through a stainless mesh, and suspended in an Eagle's minimum essential medium (MEM) to separate antibody producing cells.

### (C) Preparation of myeloma cells (hereinafter, referred to as "myeloma")

The myeloma cells to be used for cellular fusion are not particularly limited, and can be selected from known cell lines. However, considering convenience in selection of hybridoma from fused cells, it is preferable to use HGPRT (Hypoxanthine-guanine phosphoribosyl transferase)-deficient lines for which selection procedures have been established.

Specifically, examples thereof include mouse-derived X63-Ag8 (X63), NS1-ANS/1 (NS1), P3X63-Ag8.U1 (P3U1), X63-Ag8.653 (X63.653), SP2/0-Ag14 (SP2/0), MPC11-45.6TG1.7 (45.6TG), FO, S149/5XXO and BU.1, rat-derived 210.RSY3.Ag.1.2.3(Y3), and human-derived U266AR (SKO-007), GM1500-GTG-A12 (GM1500), UC729-6, LICR-LOW-HMy2 (HMy2) and 8226AR/NIP4-1 (NP41).

These HGPRT-deficient lines can be acquired from, for example, American Type Culture Collection (ATCC), etc.

These cell lines are subcultured in an appropriate medium, e.g. a 8-azaguanine medium [medium with 8-azaguanine added to a medium obtained by adding glutamine, 2-mercaptoethanol, gentamicin and fetal bovine serum (hereinafter, referred to as "FCS") to a RPMI-1640 medium], an Iscove's modified Dulbecco's medium (hereinafter, referred to as "IMDM"), or a Dulbecco's modified Eagle medium (hereinafter, referred to as "DMEM"). The cell lines are subcultured in a normal medium [e.g. ASF104 medium containing 10% FCS (manufactured by Ajinomoto Co., Inc.)] 3 to 4 days before cellular fusion, so that a cell number of 2 × 10⁷ or more is secured on the day of cellular fusion.

### (d) Cellular fusion

Fusion of antibody producing cells and myeloma cells can be appropriately performed in accordance with a known method (Weir, D.M., Handbook of Experimental Immunology Vol. I. II. III., Blackwell Scientific Publications, Oxford (1987), Kabat, E.A. and Mayer, M.M., Experimental Immunochemistry, Charles C Thomas Publisher Spigfield, Illinois (1964), etc.) and under conditions which do not cause extreme reduction of the cell viability.

Examples of the method that can be used include chemical methods in which antibody producing cells are mixed with myeloma cells in a high-concentration polymer solution of polyethylene glycol or the like; and physical methods using electric stimuli.

A specific example of the chemical methods, among the above-mentioned methods, is as follows. When polyethylene glycol is used as a high-concentration polymer solution, antibody producing cells are mixed with myeloma cells at 30 to 40°C, preferably 35 to 38°C for 1 to 10 minutes, preferably 5 to 8 minutes in a solution of polyethylene glycol having a molecular weight of 1500 to 6000, preferably 2000 to 4000.

### (e) Selection of hybridoma group

The method for selecting hybridoma obtained by the cellular fusion is not particularly limited, and a HAT (hypoxanthine aminopterin thymidine) selection method (Kohler et al., Nature (1975) 256, p.495; Milstein et al., Nature (1977) 266, p.550) is normally used.

This method is effective for obtaining hybridoma using myeloma cells of HGPRT-deficient lines which cannot survive with aminopterin.

That is, by culturing unfused cells and hybridoma in a HAT medium, only hybridoma having resistance to aminopterin can be made to remain selectively, and grown.

### (f) Division into single-cell clones (cloning)

As a method for cloning hybridoma, a known method such as a methylcellulose method, a soft agarose method or a limiting dilution method can be used (e.g. Barbara, B.M. and Stanley, M.S.: Selected Methods in Cellular Immunology, W.H. Freeman and Company, San Francisco (1980)). Among these methods, the limiting dilution method is particularly preferred.

In this method, fetal rat-derived fibroblastic cell lines, or feeders such as normal mouse spleen cells, thymus gland cells or ascites cells are inoculated in a microplate.

On the other hand, hybridoma is diluted to 0.2 to 0.5 cells/0.2 ml in the medium beforehand, 0.1 ml of the suspension of the diluted hybridoma is put in each well, and culture is continued for about 2 weeks while about one third of the medium is replaced by a fresh medium periodically (e.g. every 3 days), whereby clones of hybridoma can be grown.

For wells confirmed to have an antibody value, cloning using, for example, a limiting dilution method is repeated 2 to 4 times, and cell lines confirmed to have an antibody value with stability are selected as anti-Siglec-15 monoclonal antibody producing hybridoma lines.

Examples of the hybridoma lines cloned in the manner described above include hybridoma #32A1 and hybridoma #41B1. Hybridoma #32A1 and hybridoma #41B1 are deposited in National Institute of Advanced Industrial Science and Technology, International Patent Organisms Depositary (the current National Institute of Technology and Evaluation, Biological Resource Center, National Patent Microorganisms Depositary). Hybridoma #32A1 is labeled as anti-Siglec-15 Hybridoma #32A1 and given accession number: FERM BP-10999, and hybridoma #41B1 is labeled as anti-Siglec-15 Hybridoma #41B1 and given accession number: FERM BP-11000.

### (g) Preparation of monoclonal antibody by culturing hybridoma

By culturing the hybridoma selected in the manner described above, a monoclonal antibody can be efficiently obtained, and it is desirable that before the culture, hybridoma producing a desired monoclonal antibody be screened.

For the screening, a known method itself can be employed.

Measurement of the antibody value in the present invention can be performed by, for example, the ELISA method described in the item (b).

The hybridoma obtained by the method described above can be stored in a frozen state in a liquid nitrogen or a freezer at -80°C or lower.

The hybridoma after completion of cloning is cultured with the medium changed from the HT medium to the normal medium.

Mass culture is performed as rotary culture or spinner culture using a large culture bottle. By performing purification from the supernatant in the mass culture using a method known to those skilled in the art, such as gel filtration, a monoclonal antibody which binds specifically to the protein in the present invention can be obtained.

By intraperitoneally injecting hybridoma to a mouse in the same strain (e.g. the above-described BALB/c) or a Nu/Nu mouse, and growing the hybridoma, ascites containing a large amount of the monoclonal antibody according to the present invention can be obtained.

When the hybridoma is intraperitoneally administered, a larger amount of the ascites can be obtained by administering a mineral oil such as 2,6,10,14-tetramethyl pentadecane (pristane) beforehand (3 to 7 days before).

For example, an immunosuppressive drug is intraperitoneally injected to a mouse in the same strain as the hybridoma beforehand to inactivate T-cells, and after 20 days, 10⁶ to 10⁷ hybridoma clone cells are intraperitoneally administered in a state of being suspended in a medium (0.5 ml) which does not contain serum. When normally the abdominal part swells and ascites is accumulated, ascites is collected from the mouse.

By this method, a monoclonal antibody is obtained in a concentration that is not less than about 100 times the concentration in the culture solution.

The monoclonal antibody obtained by the above-described method can be purified using a method as described in, for example, Weir, D.M.: Handbook of Experimental Immunology, Vol. I, II, III, Blackwell Scientific Publications, Oxford (1978).

Examples of the method include an ammonium sulfate salting-out method, a gel filtration method, an ion-exchange chromatography method and an affinity chromatography method.

As a convenient method for purification, a commercially available monoclonal antibody purification kit or the like can be used.

The monoclonal antibody thus obtained has high antigenic specificity to Siglec-15.

### (h) Evaluation of monoclonal antibody

The isotypes and subclasses of the monoclonal antibody thus obtained can be determined in the following manner.

First, examples of the identification method include an Ouchterlony method, an ELISA method and a RIA method.

The Ouchterlony method is convenient, but requires a concentration operation when the concentration of the monoclonal antibody is low.

On the other hand, when the ELISA method or the RIA method is used, the culture supernatant is directly reacted with an antigen adsorption solid phase, and antibodies corresponding to various immunoglobulin isotypes and subclasses can be used as secondary antibodies to identify the isotypes and subclasses of the monoclonal antibody.

As a further convenient method, a commercially available kit for identification (e.g. Mouse Typer Kit manufactured by Bio-Rad Laboratories, Inc.) or the like can be used.

Further, the protein can be quantitatively determined by a Folin Lowry method, and a method of calculation from an absorbance at 280 nm [1.4 (OD280) = immunoglobulin 1 mg/ml].

### (3) Other antibodies

The antibodies according to the present invention include not only the monoclonal antibodies to Siglec-15 but also genetic recombinant antibodies artificially modified for reduction of heterogenous antigenicity to humans, or the like, e.g. chimeric antibodies, humanized antibodies and human antibodies. These antibodies can be produced using a known method.

Examples of the chimeric antibody include antibodies in which the variable region and the constant region of the antibody are heterogenous to each other, e.g. chimeric antibodies in which the variable region of a mouse-derived antibody is bonded to a human-derived constant region (Proc. Natl. Acad. Sci. U.S.A., 81, 6851-6855 (1984)).

Examples of the humanized antibody include antibodies in which only a complementarity determining region (CDR) is incorporated in a human-derived antibody (Nature (1986) 321, p.522-525), and antibodies in which in addition to sequences of CDR, amino acid residues of some frameworks are implanted into a human antibody by CDR implant method (WO 90/07861).

Further, examples of the antibody according to the present invention include human antibodies. The anti-Siglec-15 human antibody means a human antibody having only a gene sequence of a human chromosome-derived antibody. The anti-Siglec-15 human antibody can be acquired by a method using a human antibody producing mouse having human chromosome fragments containing genes of the H-chain and the L-chain of the human antibody (Tomizuka, K. et al., Nature Genetics (1997) 16, p.133-143; Kuroiwa, Y. et. al., Nuc. Acids Res. (1998)26, p.3447-3448; Yoshida, H. et. al., Animal Cell Technology: Basic and Applied Aspects vol. 10, p.69-73 (Kitagawa, Y., Matuda, T. and Iijima, S. eds), Kluwer Academic Publishers, 1999; Tomizuka, K. et. al., Proc. Natl. Acad. Sci. USA (2000) 97, p. 722-727).

Such transgenic animals can be generated specifically by producing knockout animals and transgenic animals in which the gene loci of the endogenous immunoglobulin heavy chain and light chain of a non-human mammal are destroyed and instead, the gene loci of the human immunoglobulin heavy chain and light chain are introduced, and crossing of these animals.

By a genetic recombination technique, eukaryotic cells are transformed with cDNA encoding each of the heavy chain and the light chain of the human antibody, preferably a vector containing the cDNA, and the transformed cells which produce a genetically modified human monoclonal antibody are cultured, whereby the monoclonal antibody can be obtained from the culture supernatant.

Here, as the host, for example, eukaryotic cells, preferably mammal cells such as CHO cells, lymphocytes and myeloma can be used.

Further, methods for acquiring a phage display-derived human antibody selected from a human antibody library (Wormstone, I.M. et al., Investigative Ophthalmology & Visual Science. (2002) 43(7), p.2301-2308; Carmen, S. et al., Briefings in Functional genomics and Proteomics (2002), 1(2), p.189-203; Siriwardena, D. et al., Opthalmology (2002) 109(3), p.427-431) are known.

For example, a phage display method can be used in which the variable region of a human antibody is expressed as a single-chain antibody (scFv) on a phage surface, and a phage binding to an antigen is selected (Nature Biotechnology (2005), 23, (9), p.1105-1116).

By analyzing a gene of the phage selected as binding to the antigen, a DNA sequence which encodes the variable region of a human antibody binding to the antigen can be determined.

When the DNA sequence of scFv binding to the antigen is revealed, a human antibody can be acquired by preparing an expression vector having the sequence, and introducing the expression vector into an appropriate host to induce expression (WO 92/01047, WO 92/20791, WO 93/06213, WO 93/11236, WO 93/19172, WO 95/01438, WO 95/15388, Annu. Rev. Immunol (1994) 12, p.433-455, Nature Biotechnology (2005) 23(9), p.1105-1116).

When an antibody gene is isolated once, and then introduced into an appropriate host to prepare an antibody, a combination of the appropriate host and an expression vector can be used.

When eukaryotic cells are used as a host, animal cells, plant cells and eukaryotic microorganisms can be used.

Examples of the animal cells include (1) mammal cells, e.g. COS cells which are cells of monkeys (Gluzman, Y. Cell (1981) 23, p.175-182, ATCC CRL-1650), mouse fibroblastic cells NIH3T3 (ATCC No. CRL-1658), and dihydrofolate reductase-deficient lines (Urlaub, G. and Chasin, L. A. Proc. Natl. Acad. Sci. U.S.A. (1980) 77, p.4126-4220) of Chinese hamster ovary cells (CHO cells, ATCC CCL-61).

When eukaryotic cells are used, examples thereof include Escherichia coli and Bacillus subtilis.

A target antibody gene is introduced into these cells by transformation, and the transformed cells are cultured *in vitro* to obtain an antibody.

The isotype of the antibody in the present invention is not limited, and examples thereof include IgG (IgG1, IgG2, IgG3, IgG4), IgM, IgA (IgA1, IgA2), IgD and IgE. The isotype is preferably IgG or IgM, more preferably IgG2.

The antibody according to the present invention may be an antibody functional fragment having an antigen-binding site of the antibody, or a modified product thereof. By treating an antibody with a proteolytic enzyme such as papain or pepsin, or modifying an antibody gene by a genetic engineering method, and an antibody is expressed in appropriate cultured cells, fragments of the antibody can be obtained. Among these antibody fragments, fragments retaining all or some of functions of the overall molecules of the antibody can be called antibody functional fragments. Examples of the antibody function generally include antigen binding activity, antigen activity-neutralizing activity, antigen activity-enhancing activity, antibody-dependent cellular cytotoxic activity, complement-dependent cellular cytotoxic activity and complement-dependent cell-mediated cellular cytotoxic activity. The function retained by the functional fragments of the antibody in the present invention is preferably the activity of suppressing formation of osteoclasts, more preferably the activity of suppressing the process of cellular fusion of osteoclasts.

Examples of the fragments of the antibody include Fab, F(ab')₂, Fv, single-chain Fv(scFv) obtained by connecting Fvs of the heavy chain and the light chain with an appropriate linker, diabodies, linear antibodies, and multispecific antibodies composed of antibody fragments. The fragments of the antibody also include Fab' which is a univalent fragment of a variable region of an antibody obtained by treating F(ab')₂ under reducing conditions.

Further, the antibody according to the present invention may be a multispecific antibody having specificity to at least two different antigens.

Such a molecule normally binds two antigens (i.e. bispecific antibody), and the "multispecific antibody" in the present invention includes antibodies having specificity to two or more antigens (e.g. three antigens).

The multispecific antibody according to the present invention may be an overall antibody, or a fragment of such an antibody (e.g. F(ab')₂ bispecific antibody). The bispecific antibody can be prepared either by binding the heavy chains and the light chains (HL pairs) of two antibodies, or by fusing hybridomas, which produce different monoclonal antibodies, to prepare bispecific antibody producing fused cells (Millstein et al., Nature (1983) 305, p.537-539).

The antibody according to the present invention may be a single-chain antibody (also referred to as scFv). The single-chain antibody is obtained by connecting the heavy chain V region and the light chain V region of the antibody with a linker of a polypeptide (Pluckthun, The Pharmacology of Monoclonal Antibodies, 113 (edited by Rosenburg and Moore, Springer Verlag, New York, p.269-315 (1994), Nature Biotechnology (2005), 23, p.1126-1136). Further, a BiscFv fragments prepared by binding two scFvs with a polypeptide linker can be used as a bispecific antibody.

Methods for preparing a single-chain antibody are well known in the art (see, for example, U.S. Patent Nos. 4,946,778, 5,260,203, 5,091,513 and 5,455,030). In the scFv, the heavy chain V region and the light chain V region are connected through a linker which does not form a conjugate, preferably a polypeptide linker (Huston, J.S. et al., Proc. Natl. Acad. Sci. U.S.A. (1988), 85, p.5879-5883). The heavy chain V region and the light chain V region in scFv may be derived from the same antibody, or derived from different antibodies. As a polypeptide linker which connects V regions, for example, any single-chain peptide consisting of 12 to 19 residues is used.

A DNA part which encodes all or desired amino acid sequences of the sequences of DNA which encodes the heavy chain or the heavy chain V region and DNA which encodes the light chain or the light chain V region of the antibody is set to a template, and amplified by a PCR method using a primer pair defining both ends of the DNA part, and then further amplified by a combination of DNA which encodes a polypeptide linker part and a primer pair defining both ends of the DNA so as to connect the ends to the heavy chain and the light chain, respectively, whereby DNA which encodes scFv is obtained.

When DNA which encodes scFv is once prepared, an expression vector containing the DNA and a host transformed by the expression vector can be obtained in accordance with a conventional method, and by using the host, scFv can be obtained in accordance with a conventional method.

These antibody fragments can be expressed by acquiring a gene, and produced by a host in the same manner as described above.

The antibody according to the present invention may be polymerized to enhance its affinity to an antigen. One antibody, or a plurality of antibodies which recognize a plurality of epitopes of one antigen may be polymerized. Examples of the method for polymerizing the antibody include binding of an IgG CH3 domain with two scFvs, binding with streptavidin, and introduction of a Helix-turn-helix motif.

The antibody according to the present invention may be a polyclonal antibody which is a mixture of a plurality of kinds of anti-Siglec-15 antibodies having different amino acid sequences. Examples of the polyclonal antibody include mixtures of a plurality of kinds of antibodies having different CDRs. As such a polyclonal antibody, an antibody purified from a cultured product obtained by culturing a mixture of cells which produce different antibodies can be used (see WO 2004/061104).

As modified products of antibodies, antibodies bound to various molecules such as polyethylene glycol (PEG) can be used.

The resulting antibody can be purified so that the antibody becomes uniform. Separation and purification of the antibody may be performed by a separation and purification method which is used for normal proteins.

By appropriately selecting and combining, for example, a chromatography column, a filter, ultrafiltration, salting-out, dialysis, polyacrylamide gel electrophoresis for preparation, isoelectric point electrophoresis and the like, the antibody can be separated and purified (Strategies for Protein Purification and Characterization: A Laboratory Course Manual, Daniel R. Marshak et al. eds., Cold Spring Harbor Laboratory Press (1996); Antibodies: A Laboratory Manual. Ed Harlow and David Lane, Cold Spring Harbor Laboratory (1988)). However, the separation and purification method is not limited thereto.

Examples of the chromatography include affinity chromatography, ion-exchange chromatography, hydrophobic chromatography, gel filtration chromatography, reverse phase chromatography and adsorption chromatography.

Such chromatography can be carried out by liquid chromatography such as HPLC or FPLC.

Examples of the column to be used for affinity chromatography include protein A columns and protein G columns.

Examples of the column using a protein A column include POROS, and Sepharose F.F.

Using a carrier on which an antigen is immobilized, the antibody can be purified by means of a binding property to the antigen.

Preferably, the anti-Siglec-15 antibody in the present invention is an antibody having activity of suppressing formation of osteoclasts and/or bone resorption by osteoclasts.

The activity of the anti-Siglec-15 antibody can be evaluated by *in vitro* measurement of the activity of suppressing differentiation of cells, which excessively express Siglec-15, into osteoclasts. For example, the anti-Seglec-15 antibody can be added in various concentrations to mouse monocyte-derived cell line RAW264.7 cells or RAW264 cells to measure the activity of suppressing differentiation into osteoclasts by the RANKL (receptor activator of NF-KB) or TNF-α stimulus. Further, the anti-Siglec-15 antibody can be added in various concentrations to bone marrow-derived primary cultured cells to measure the activity of suppressing differentiation into osteoclasts by the RANKL, TNF-α or active vitamin D₃ stimulus. Further, the anti-Siglec-15 antibody can be added in various concentrations to normal human osteoclastic precursor cells to measure the activity of suppressing differentiation into osteoclasts by the RANKL and M-CSF stimuli. Such a suppressing effect on differentiation into osteoclasts can be measured with suppression of tartaric acid resistance acidic phosphatase (TRACP) activity of osteoclasts as an indicator. Further, the suppressing effect on differentiation into osteoclasts can be measured with suppression of formation of TRACP positive multinucleate osteoclasts, i.e. suppression of cellular fusion of osteoclasts, as an indicator. For example, it is possible to select an antibody exhibiting a suppressing effect on cellular fusion at a concentration of 30 µg/ml or less or at a concentration of 3 µg/ml or less or 1 µg/ml or less in the system for testing differentiation into osteoclasts. Further, when the test is conducted on the effect at a lower concentration, an antibody exhibiting a suppressing effect on differentiation into osteoclasts over the concentration range of 63 ng/ml to 1 µg/ml may be selected. Further, in pit assay (Takada et al., Bone and Mineral (1992) 17, 347-359) experiments using cells derived from the femur and/or the tibia, the activity of suppressing bone resorption by osteoclasts *in vitro* can be measured by adding the anti-Siglec-15 antibody in various concentrations to the cells derived from the femur and/or the tibia, and observing formation of pits on the ivory section. Further, as a system for measuring the activity of suppressing bone resorption by osteoclasts *in vitro,* a plate coated with human collagen to which europium binds can be used (WO 2009/048072, Example 37). For example, it is possible to select an antibody exhibiting a suppressing effect on bone resorption at a concentration of 3 µg/ml or less, i.e. over the concentration range of 0.3 µg/ml to 3 µg/ml in the system for testing bone resorption by osteoclasts. On the other hand, when an experimental animal is used *in vivo,* the activity of the anti-Siglec-15 antibody can be confirmed by measuring a change of osteoclasts in a secondary spongiosa region.

Examples of the anti-Siglec-15 antibody that can be used in the present invention include anti-Siglec-15 antibodies disclosed in WO 2009/048072, WO 2010/117011, WO 2013/147212, WO 2013/147213, WO 2012/045481, etc. Examples of the anti-Siglec-15 antibody that can be used in the present invention include antibodies which is produced by the hybridoma #32A1 (FERM BP-10999) (hereinafter, referred to as "#32A1 antibodies"), and the #32A1 antibody has a heavy chain variable region consisting of an amino acid sequence consisting of amino acid residues at positions 20 to 140 in the amino acid sequence of SEQ ID NO: 21 and a light chain variable region consisting of an amino acid sequence consisting of amino acid residues at positions 21 to 132 in the amino acid sequence of SEQ ID NO: 22. Further, examples of the anti-Siglec-15 antibody that can be used in the present invention include monoclonal antibodies which compete with the #32A1 antibody or have a common epitope in binding to Siglec-15, and suppress formation of osteoclasts and/or bone resorption by osteoclasts, characterized in that bone resorption by osteoclasts *in vitro* is suppressed at a concentration of 3µg/ml or less. The epitope of the #32A1 antibody is a human Siglec-15 V-set domain (domain consisting of amino acid residues at positions 39 to 165 in the amino acid sequence of accession number: NP_998767 in the NCBI protein database or the amino acid sequence set forth as SEQ ID NO: 2 in Sequence Listing).

The anti-Siglec-15 antibody that can be used in the present invention is preferably a humanized antibody of the #32A1 antibody, or a CDR-modified product thereof. Examples of the humanized antibody of the #32A1 antibody include combinations of a heavy chain containing a heavy chain variable region consisting of an amino acid sequence consisting of amino acid residues at positions 20 to 140 in the amino acid sequence of SEQ ID NO: 7 and a light chain containing a light chain variable region consisting of an amino acid sequence consisting of amino acid residues at positions 21 to 133 in the amino acid sequence of SEQ ID NO: 8; combinations of a heavy chain containing a heavy chain variable region consisting of an amino acid sequence consisting of amino acid residues at positions 20 to 140 in the amino acid sequence of SEQ ID NO: 9 and a light chain containing a light chain variable region consisting of an amino acid sequence consisting of amino acid residues at positions 21 to 133 in the amino acid sequence of SEQ ID NO: 8; combinations of a heavy chain containing a heavy chain variable region consisting of an amino acid sequence consisting of amino acid residues at positions 20 to 140 in the amino acid sequence of SEQ ID NO: 9 and a light chain containing a light chain variable region consisting of an amino acid sequence consisting of amino acid residues at positions 21 to 133 in the amino acid sequence of SEQ ID NO: 10; and combinations of a heavy chain containing a heavy chain variable region consisting of an amino acid sequence consisting of amino acid residues at positions 20 to 140 in the amino acid sequence of SEQ ID NO: 9 and a light chain containing a light chain variable region consisting of an amino acid sequence consisting of amino acid residues at positions 21 to 133 in the amino acid sequence of SEQ ID NO: 11.

Examples of the more preferred humanized antibody include combinations of a heavy chain containing a heavy chain variable region consisting of an amino acid sequence consisting of amino acid residues at positions 20 to 466 in the amino acid sequence of SEQ ID NO: 7 and a light chain containing a light chain variable region consisting of an amino acid sequence consisting of amino acid residues at positions 21 to 238 in the amino acid sequence of SEQ ID NO: 8; combinations of a heavy chain containing a heavy chain variable region consisting of an amino acid sequence consisting of amino acid residues at positions 20 to 466 in the amino acid sequence of SEQ ID NO: 9 and a light chain containing a light chain variable region consisting of an amino acid sequence consisting of amino acid residues at positions 21 to 238 in the amino acid sequence of SEQ ID NO: 8; combinations of a heavy chain containing a heavy chain variable region consisting of an amino acid sequence consisting of amino acid residues at positions 20 to 466 in the amino acid sequence of SEQ ID NO: 9 and a light chain containing a light chain variable region consisting of an amino acid sequence consisting of amino acid residues at positions 21 to 238 in the amino acid sequence of SEQ ID NO: 10; and combinations of a heavy chain containing a heavy chain variable region consisting of an amino acid sequence consisting of amino acid residues at positions 20 to 466 in the amino acid sequence of SEQ ID NO: 9 and a light chain containing a light chain variable region consisting of an amino acid sequence consisting of amino acid residues at positions 21 to 238 in the amino acid sequence of SEQ ID NO: 11.

However, the humanized antibody of the #32A1 antibody is not limited to the above-described humanized antibodies as long as it retains all of the six CDR sequences of the #32A1 antibody, and has activity of suppressing formation of osteoclasts and/or bone resorption by osteoclasts. The heavy chain variable region of the #32A1 antibody has CDRH1 (DYFMN) consisting of the amino acid sequence set forth as SEQ ID NO: 12, CDRH2 (QIRNKIYTYATFYA) consisting of the amino acid sequence set forth as SEQ ID NO: 13, and CDRH3 (SLTGGDYFDY) consisting of the amino acid sequence set forth as SEQ ID NO: 14. The light chain variable region of the #32A1 antibody has CDRL1 (RASQSVTISGYSFIH) consisting of the amino acid sequence set forth as SEQ ID NO: 15, CDRL2 (RASNLAS) consisting of the amino acid sequence set forth as SEQ ID NO: 16, and CDRL3 (QQSRKSPWT) consisting of the amino acid sequence set forth as SEQ ID NO: 17.

Examples of the CDR-modified product of the humanized antibody of the #32A1 antibody include humanized antibodies of the #32A1 antibody in which the 3rd threonine residue of CDRH3 of SEQ ID NO: 14 is substituted with a glutamic acid residue. Siglec-15 is a basic protein, and is expected to have a binding ability improved with an antigen-antibody ionic bond formed by introduction of acidic amino acid residues such as asparagine acid and glutamic acid into an antibody sequence. A substitution product has been designed in which a glutamic acid residue that is an acidic amino acid and has a long side chain is introduced at a threonine residue positioned at the center of the CDRH3 loop which is considered most important of antibody recognition sites and which is predicted to be oriented to the antigen side in X-ray crystallography. The CDRH3 (SLEGGDYFDY) substituted as described above corresponds to the amino acid sequence set forth as SEQ ID NO: 18 in Sequence Listing.

Examples of the CDR-modified product include combinations of a heavy chain containing a heavy chain variable region consisting of an amino acid sequence consisting of amino acid residues at positions 20 to 140 in the amino acid sequence set forth as SEQ ID NO: 19 and a light chain containing a light chain variable region consisting of an amino acid sequence consisting of amino acid residues at positions 21 to 133 in the amino acid sequence set forth as SEQ ID NO: 20.

Examples of the more preferred CDR-modified product include an antibody consisting of a heavy chain having an amino acid sequence consisting of amino acid residues at positions 20 to 466 in the amino acid sequence set forth as SEQ ID NO: 19 and a light chain having an amino acid sequence consisting of amino acid residues at positions 21 to 238 in the amino acid sequence set forth as SEQ ID NO: 20.

However, the CDR-modified product of the humanized antibody of the #32A1 antibody is not limited to the above-described CDR-modified products as long as it has the CDRH3 sequence of SEQ ID NO: 18, and has activity of suppressing formation of osteoclasts and/or bone resorption by osteoclasts.

It is known that a lysine residue at the heavy chain carboxyl end of an antibody produced in cultured mammal cells is deleted (Journal of Chromatography A, 705: 129-134 (1995)), and it is known that two amino acid residues: glycine and lysine at the heavy chain carboxyl end are also deleted, and additionally a proline residue positioned at the carboxyl end is amidated (Analytical Biochemistry, 360: 75-83 (2007)). However, the deletion and the modification in the heavy chain sequence have no effect on the antigen binding ability and the effector function (complement activation and antibody-dependent cellular cytotoxicity) of the antibody. Therefore, the present invention includes antibodies which have undergone such modifications, and examples thereof include deletion products in which one or two amino acids are deleted at the heavy chain carboxyl end, and the deletion products which are amidated (e.g. heavy chains in which a proline residue at the carboxyl end site is amidated). However, the deletion product of the antibody according to the present invention in which deletion occurs at the heavy chain carboxyl end is not limited to the above-mentioned types, as long as the antigen binding ability and the effector function are retained. Two heavy chains forming the antibody according to the present invention may be any one of heavy chains selected from the group consisting of the full-length heavy chain and the above-described deletion products, or a combination of any two thereof. The mass ratio of each deletion product may be affected by the type and the culture conditions of cultured mammal cells which produce the antibody according to the present invention, and examples of the main component of the antibody according to the present invention include those in which one amino acid residue at the carboxyl end is deleted in both of the two heavy chains.

### 3. Pharmaceutical agent containing anti-Siglec-15 antibody

The anti-Siglec-15 antibody can be used as an active ingredient of a pharmaceutical agent for the treatment and/or prophylaxis of pediatric osteoporosis.

Pediatric osteoporosis means osteoporosis which is developed growing children (about 17 years old or younger). Causes of pediatric osteoporosis are various, and there are pediatric osteoporosis caused by dysosteogenesis or idiopathic pediatric osteoporosis (classified as primary osteoporosis), and pediatric osteoporosis caused by nerve disease, endocrine/inflammatory disease, blood disease or drug administration (classified as secondary osteoporosis). In the present invention, the "pediatric osteoporosis" is preferably pediatric osteoporosis which is developed due to drug administration. Examples of the drug which causes pediatric osteoporosis include, but are not limited to, steroid drugs such as methylprednisolone and prednisolone, and immunosuppressive drugs such as tacrolimus, cyclosporin and methotrexate. In the present invention, the "pediatric osteoporosis" is more preferably pediatric steroid-induced osteoporosis which is developed due to administration of a steroid drug. Since children are at the age where bone formation and growth is significant, and they are easily affected by an existing potent bone resorption inhibitor such as a bisphosphonate preparation which is administered for treatment of osteoporosis. Thus, pediatric osteoporosis is a disease in which severe growth disorder, abnormal bone structure and abnormal bone substance may be developed when treatment is performed with a bone resorption inhibitor.

In the present invention, the anti-Siglec-15 antibody can be provided in the form of a pharmaceutical composition with a pharmaceutically acceptable diluent, carrier, solubilizer, emulsifier, preservative and/or adjuvant. The pharmaceutical composition may contain a therapeutically and/or prophylactically effective amount of the anti-Siglec-15 antibody.

Substances to be used for the preparation acceptable in the pharmaceutical composition of the present invention are preferably nontoxic to a person who is given the pharmaceutical composition, in terms of a dose and a dosing concentration.

The pharmaceutical composition of the present invention may contain substances for preparations for changing or maintaining the pH, osmotic pressure, viscosity, transparency, color, isotonicity, aseptic property, stability, dissolution rate, sustained-releasability, absorption ratio and penetration rate. Examples of the substances for preparations include, but are not limited to, amino acids such as glycine, alanine, glutamine, asparagine, arginine and lysine; antibacterial agents; antioxidants such as ascorbic acid, sodium sulfate and sodium hydrogensulfite; buffers such as phosphoric acid, citric acid, borate buffers, sodium hydrogencarbonate and tris-hydrochloric acid (Tris-HCl) solutions; fillers such as mannitol and glycine; chelating agents such as ethylenediaminetetraacetic acid (EDTA); complexing agents such as caffeine, polyvinylpyrrolidine and β-cyclodextrin and hydroxypropyl-β-cyclodextrin; extenders such as glucose, mannose and dextrin; other carbohydrates such as monosaccharides and disaccharides; colorants; flavors; diluents; emulsifiers; hydrophilic polymers such as polyvinylpyrrolidine; preservatives such as low-molecular-weight polypeptides, salt-forming counter ions, benzalkonium chloride, benzoic acid, salicylic acid, thimerosal, phenethyl alcohol, methylparaben, propylparaben, chlorhexidine, sorbic acid and hydrogen peroxide; solvents such as glycerin, propylene glycol and polyethylene glycol; sugar alcohols such as mannitol and sorbitol; suspension agents; surfactants such as sorbitan esters, polysorbates such as polysorbate 20 and polysorbate 80, triton, tromethamine, lecithin and cholesterol; stability enhancing agents such as sucrose and sorbitol; elasticity enhancing agents such as sodium chloride, potassium chloride and mannitol/sorbitol; transport agents; diluents; excipients; and/or pharmaceutical adjuvants. Preferably, these substances for preparations are added in an amount 0.01 to 100 times, particularly 0.1 to 10 times the weight of the anti-Siglec-15 antibody. The preferred composition of the pharmaceutical composition in the preparation can be appropriately determined according to an applicable disease, an applicable administration route or the like by those skilled in the art.

The excipient or carrier in the pharmaceutical composition may be either liquid or solid. The appropriate excipient or carrier may be water and physiological saline for injection, an artificial cerebrospinal fluid, or another substance that is normally used for parenteral administration. Neutral physiological saline, or physiological saline containing serum albumin can be used for the carrier. The pharmaceutical composition may contain a Tris buffer having a pH of 7.0 to 8.5, an acetate buffer having a pH of 4.0 to 5.5, or such a buffer containing sorbitol or other compounds. As a drug having a selected composition and a necessary purity, the pharmaceutical composition of the present invention is prepared as a freeze-dried product or a liquid. The pharmaceutical composition of the present invention can be formed as a freeze-dried product using an appropriate excipient such as sucrose.

The pharmaceutical composition of the present invention can be prepared for parenteral administration, or prepared for absorption into the gastrointestinal tract through oral administration. The composition and the concentration of the preparation can be determined according to an administration method, and the dose to a human, at which drug potency is exhibited, can be reduced as the affinity of the anti-Siglec-15 antibody, which is contained in the pharmaceutical composition of the present invention, to Siglec-15, is enhanced, i.e. the dissociation constant (Kd value) against Siglec-15 decreases. Therefore, on the basis of the results from these considerations, the dose of the pharmaceutical composition of the present invention to a human can be determined. The human anti-Siglec-15 antibody may be administered to a human once every 1 to 180 days at a dose of about 0.1 to 100 mg/kg.

Examples of the form of the pharmaceutical composition of the present invention include injections including drip infusions, suppositories, intranasal formulations, sublingual formulations and transdermal formulations.

The pharmaceutical composition of the present invention may contain, in addition to the anti-Siglec-15 antibody, one or more ingredients effective for treatment and/or prevention of bone disease. Examples of the ingredient include, but are not limited to, active vitamin D₃, calcitonin and derivatives thereof, hormone preparations such as estradiol, SERMs (selective estrogen receptor modulators), ipriflavone, vitamin K₂ (menatetrenone), calcium preparations, PTH (parathyroid hormone) preparations, non-steroid anti-inflammatory drugs, soluble TNF receptor preparations, anti-TNFα antibodies or functional fragments of the antibodies, anti-PTHrP (parathyroid hormone-related protein) antibodies or functional fragments of the antibodies, IL-1 receptor antagonists, and anti-IL-6 receptor antibodies or functional fragments of the antibodies.

The ingredients may be incorporated in the same preparation as that of the anti-Siglec-15 antibody, or incorporated in a preparation different from that of the anti-Siglec-15 antibody, and supplied together with or separately from the anti-Siglec-15 antibody. Alternatively, the ingredients may be supplied in a state of being bound to the anti-Siglec-15 antibody or a functional fragment thereof. The anti-Siglec-15 antibody or a functional fragment thereof can be bound with the ingredients in various binding modes described in, for example, M.C. Garnet "Targeted drug conjugates: principles and progress", Advanced Drug Delivery Reviews, (2001) 53, 171-216, G.T. Hermanson "Bioconjugate Techniques" Academic Press, California (1996), Putnam and J. Kopecek "Polymer Conjugates with Anticancer Activity" Advances in Polymer Science (1995) 122, 55-123.

### Example

Hereinafter, the present invention will be described in detail by way of Example, which should not be construed as limiting the present invention. A. Evaluation using growing healthy rat

### I. Experimental method

### (1) Animals used

6-week-old growing male F344 rats were used.

### (2) Experimental groups (n=10 in each group)

(i) Control group (hereinafter, referred to as "Ctl group")
(ii) Anti-Siglec-15 antibody administration group (hereinafter, referred to as "Sig-15 Ab administration group"): The #32A1 antibody was used as an anti-Siglec-15 antibody. The anti-Siglec-15 antibody was subcutaneously administered once every 3 weeks at a dosage of 0.25, 1 or 4 mg/kg.
(iii) Bisphosphonate administration group (hereinafter, referred to as "ALN administration group"): Alendronate (ALN) (manufactured by LKT Laboratories, Inc.) was subcutaneously administered twice a week at a dosage of 0.028 or 0.140 mg/kg.

The dose of each drug was adjusted on the basis of the result of body weight measurement performed once a week.

### (3) Administration and observation period

6 weeks after the start of administration (age of 6 weeks to age of 12 weeks). After the end of the period (age of 12 weeks), the animals were euthanized, and evaluation was performed.

The rats of each experimental group were bred with normal feedstuff in a specific-pathogen free (SPF) environment. The rats were allowed to freely access feed and water.

For bone labeling, calcein was administered 7 days before and 3 days before euthanasia (at an interval of 4 days). The calcein was dissolved in a 1.4% sodium bicarbonate solution at a concentration of 10 mg/ml, and subcutaneously injected to each animal at a dosage of 10 mg/kg.

### (4) Evaluation items

### <Longitudinal evaluation>

### (i) Head trunk length and body weight

The head trunk length was measured 0, 3 and 6 weeks after administration. The body weight was measured once a week.

### (ii) Femur length

The femur length was measured under anesthesia every 3 weeks by micro-CT imaging.

### (iii) Bone formation marker and bone resorption marker

Before the start of administration, and 6 weeks after administration and before euthanasia, blood was collected from the tail vein, and the values of a bone formation marker (serum osteocalcin) and a bone resorption marker (serum TRACP-5b) were measured by an ELISA method.

Various operations were carried out in accordance with the schedule described in Figure 1.

### <Evaluation after isolation of specimens>

The animals were euthanized, and then dissected to extract the femur, the tibia and the lumber vertebra as evaluation samples.

### (i) Bone morphometry

Micro-CT imaging of the femur, the tibia and the fifth lumber vertebra was performed, and the right femur major axis length was measured.

### (ii) Histological studies

Non-decalcified hard tissue sample: a coronal section tissue of proximal left tibia 1/2 (length: about 1.5 cm) was used. The tissue was immersed and fixed in 70% ethanol, and then stored in a cool and dark place. The resulting non-decalcified hard tissue sample was used for Villanueva staining, bright-field observation and fluorescent observation, and quantitative bone morphometry.

Decalcified tissue sample: knee joint disarticulation was performed, and samples of a coronal section tissue of the proximal tibia (proximal right knee tibia 1/2) and a fifth lumber vertebra coronal section tissue were prepared.

With the non-decalcified hard tissue sample and the decalcified tissue sample, the growth cartilage plate width and the longitudinal growth rate were histologically measured to evaluate growth disorder ("Modern Bone Histomorphometry", 2014, WENET, Inc.).

Osteoclastic cells were detected and evaluated by chemical staining with tartrate-resistant acid phosphatase (TRACP) (hereinafter, referred to as "TRACP staining") and counter staining with methyl green. The growth cartilage part was detected and evaluated by safranine O staining (staining of acidic mucopolysaccharide).

### (iii) Mechanical test

The second, third, fourth and sixth lumber vertebral bodies and the distal metaphysis of the left femur were subjected to a compression test, and the ultimate load (maximum load endured before fracture), stiffness (resistance to deformation) and toughness (energy required before fracture) were evaluated.

### (iv) Measurement of bone mineral density

BMD measurement of the lumber vertebra (first to third lumber vertebrae) and the left distal femur end was performed by dual energy X-ray absorptiometric (DXA) method using a bone mineral density measuring apparatus (manufactured by Hitachi Aloka Medical, Ltd.).

### II. Experimental results

### (1) Effect of drug on head trunk length and body weight

Figure 2 shows the results of longitudinally measuring the head trunk length and the femur length after the start of the administration and observation period. At the end of the administration and observation period (at the age of 12 weeks), the Sig-15 Ab administration group was not significantly different in head trunk length and femur length from the Ct1 group.
On the other hand, the head trunk length and the femur length decreased in the ALN administration group as compared to the Ct1 group (Figure 2(A)). The same tendency was observed for the amount of change in head trunk length and femur length over the administration and observation period (Figure 2(B)).

These results show that unlike bisphosphonate, administration of the anti-Siglec-15 antibody does not cause growth disorder in a subject to be medicated.

### (2) Effect of drug on bone metabolism (bone formation marker and bone resorption marker)

Figure 3 shows the results of measuring a bone formation marker (serum osteocalcin) and a bone resorption marker (serum TRACP-5b) in a blood sample collected before and after the start of the administration and observation period. The serum TRACP-5b level at the end of the administration and observation period (at the age of 12 weeks) decreased depending on the dosage of the administered drug in both the Sig-15 Ab administration group and the ALN administration group (Figure 3(A)). On the other hand, either of these administration groups was not significantly different in serum osteocalcin level from the Ct1 group. The same tendency was observed for the amount of change over the administration and observation period (Figure 3(B)).

These results show that bone resorption is suppressed depending on the dosage in both administration of the anti-Siglec-15 antibody and administration of bisphosphonate.

### (3) Histological evaluation of effect of drug on growth

Figures 4-1 and 4-2 show the results of histologically evaluating the effect of the drug on growth for the proximal tibia at the end of the administration and observation period (at the age of 12 weeks). Figure 4-1(A) shows coronal cross-section photographs of images (3D-CT images) obtained by performing micro-CT imaging of the proximal tibia, and three-dimensionally reconstructing the acquired data. Any of the animals of the Sig-15 Ab administration group did not show a significant change as compared to the Ct1 group. On the other hand, in the ALN administration group, particularly the high-dosage administration group, cup-like shape (i.e. a small amount of change in bone thickness as going from the proximal part to the distal side) was presented (the normal shape is a trumpet-like shape), and the growth cartilage plate width (arrowhead) decreased.

Figure 4-1(C) shows the results of observing the growth cartilage and a primary spongiosa region immediately below the growth cartilage using safranine O-stained sample (staining of acidic mucopolysaccharide) which is used for evaluation of the cartilage. The width of the growth cartilage decreased in the ALN administration group as in the case of the 3D-CT image. Further, regarding safranine O staining positive (red) regions present in the bone in the primary spongiosa region, the Siglec-15 antibody administration group was not different from the Ct1 group, whereas in the ALN administration group, the regions expanded. Further, detailed observation of the safranine O staining positive growth cartilage regions revealed that in the Ct1 group and the Siglec-15 antibody administration group, the regions were orderly arranged longitudinally from a proliferated layer to an hypertrophic chondrocyte layer to a calcified cartilage cell layer, whereas in the ALN administration group, the regions tended to have a disordered arrangement.

Figure 4-1(B) shows the results of preparing a non-decalcified tissue sample of the proximal tibia obtained by labeling with calcein 7 days before and 3 days before euthanasia, and performing Villanueva staining. There are two parts labeled in parallel to the growth cartilage, the proximal labeled part (arrow (upper side)) represents the region labeled 3 days before euthanasia, and the more distal part (arrow (lower side)) represents the region labeled 7 days before euthanasia. The bone growth rate was evaluated on the basis of a distance between the two labeled regions. As a result, it was shown that the distance in the Sig-15 Ab administration group was not different from the distance in the Ct1 administration group, and thus there was no difference between the bone growth rates of these groups. On the other hand, the distance in the ALN administration group (particularly high-dosage administration group) was smaller than the distance in the Ct1 administration group, and thus the ALN administration group had a low bone growth rate.

Figure 4-2(D) shows the results of observing a primary spongiosa region of the proximal tibia using a TRACP-stained sample which is used for evaluation of osteoclasts. The number of TRACP positive cells in the Siglec-15 antibody administration group was not different from the number of the cells in the Ct1 group, whereas the number of TRACP positive cells in the ALN administration group evidently decreased.

Further, Figures 4-2(E) and 4-2(F) show the results of quantitatively evaluating the bone growth rate, the growth cartilage width, and the ratio of the osteoclast surface to the bone surface in the primary spongiosa region (Oc. Pm/B. Pm (%)). The Siglec-15 antibody administration group was not significantly different in any of the bone growth rate, the growth cartilage width and the osteoclast surface from the Ct1 group. On the other hand, the ALN administration group (particularly high-dosage administration group) was significantly inferior in all the bone growth rate, the growth cartilage width and the osteoclast surface in the primary spongiosa region to the Ct1 group.

The above results indicate that bone resorption immediately below the growth cartilage has an important role for growth of long bones, and ALN administration inhibits the bone resorption, so that the normal growth and modeling processes of bones are hindered. On the other hand, it is indicated that Siglec-15 antibody administration does not inhibit bone resorption in the region, and therefore does not affect bone growth.

### (4) Effect of drug on bone mass and mechanical strength

Figures 5-1 and 5-2 show the results of evaluating and examining the effect of drug administration using lumber vertebrae rich in trabecular bone. Figure 5-1(A) shows lumber vertebra coronal cross-section photographs obtained from 3D-CT images prepared by performing micro-CT imaging of the lumber vertebra at the end of the administration and observation period (at the age of 12 weeks). The trabecular bone mass increased depending on the dosage of the administered drug in both the Sig-15 Ab administration group and the ALN administration group as compared to the Ct1 group. In the ALN administration group, the bone mass in the primary spongiosa particularly increased.

The Figure 5-2(C) shows the results of measuring the bone mineral density of the lumber vertebra using a DXA method. The BMD value of the lumber vertebra increased depending on the dosage of the administered drug in both the Sig-15 Ab administration group and the ALN administration group as compared to the Ct1 group.

Figure 5-1(B) shows the results of observing osteoclasts in primary and secondary spongiosa regions by TRACP staining of lumber vertebra tissues. The number of TRACP positive cells in the secondary spongiosa region decreased in both the Sig-15 Ab administration group and the ALN administration group as compared to the Ct1 group, and the ratio of the osteoclast surface to the bone surface (Oc. Pm/B. Pm (%)) decreased in both the Sig-15 Ab administration group and the ALN administration group as compared to the Ct1 group (Figure 5-2(D)). These results indicate that bone resorption of the secondary spongiosa (remodeling bone) is inhibited in both the Sig-15 Ab administration group and the ALN administration group.

Figure 5-2(E) shows the results of measurement in a compression test of the lumber vertebra. The Sig-15 Ab administration group was not significantly different in ultimate load, stiffness and toughness from the Ct1 group. On the other hand, the ultimate load and the stiffness significantly increased in the ALN administration group (particularly high-dosage administration group) as compared to the Ct1 group. This is ascribable not only to change in trabecular bone mass but also to increase in bone mass of the primary spongiosa due to inhibition of bone resorption.

### (5) Effect of drug on long bone

Figures 6-1 and 6-2 show the results of evaluating and examining the effect of drug administration on the long bone using the proximal tibia and the distal femur metaphysis having a large amount of trabecular bone. Figure 6-1(A-1) shows femur coronal cross-section photographs obtained from 3D-CT images prepared by performing micro-CT imaging of the distal femur at the end of the administration and observation period (at the age of 12 weeks). The trabecular bone mass increased depending on the dosage of the administered drug in both the Sig-15 Ab administration group and the ALN administration group as compared to the Ct1 group. Figure 6-1(A-2) shows the results of measuring the bone mineral density of the same areas using a DXA method.
The BMD value increased depending on the dosage of the administered drug in both the Sig-15 Ab administration group and the ALN administration group as compared to the Ct1 group. From a histological point of view, the bone mass increased in a dosage-dependent manner, and in the ALN administration group (particularly high-dosage administration group), the bone trabecular thickness increased.

Figure 6-1(B-1) shows the results of observing osteoclasts in a secondary spongiosa region by TRACP staining of the proximal tibia at the age of 12 weeks. The number of TRACP positive cells decreased in both the Sig-15 Ab administration group and the ALN administration group as compared to the Ct1 group, and the ratio of the osteoclast surface to the bone surface (Oc. Pm/B. Pm (%)) decreased in both the Sig-15 Ab administration group and the ALN administration group as compared to the Ct1 group (Figure 6-1(B-2)).

Figure 6-2(C) shows the results of measurement in a compression test of the distal femur metaphysis at the age of 12 weeks. The ultimate load, stiffness and toughness increased in a dosage-dependent manner in the Sig-15 Ab administration group and the ALN administration group as compared to the Ct1 group.

As above, the anti-Siglec-15 antibody and bisphosphonate both enhance the trabecular bone mass, the bone mineral density and the mechanical properties in a subject to be medicated, and are therefore useful as drugs for treatment and prevention of osteoporosis, and in administration of the anti-Siglec-15 antibody, inhibition of bone resorption immediately below the growth cartilage (in the primary spongiosa region), which occurred in administration of bisphosphonate, and resulting hindrance of growing and modeling of bones did not occur. This result shows that the anti-Siglec-15 antibody is useful as a drug for treatment and prevention of osteoporosis particularly in growing children whose bones are remarkably growing.

### B. Evaluation using pediatric steroid-induced osteoporosis model rats

### I. Experimental method

### (1) Animals used

6-week-old growing female LEW/Cr1Crlj rats were used.

### (2) Animal model of pediatric steroid-induced osteoporosis

As rodent osteoporosis model animals, models with glucocorticoids administered to mice are often used, but strains in which the bone mass decreases due to administration of glucocorticoids as with humans are limited to two strains: Swiss Webster and FVB/N (Thiele S, et al. Bone Key Reports 3: 552 (2014)). However, even in these strains, it is considered that the bone mass does not decrease in young mice at the age of 20 weeks or less, for which bone growth has not been completed. Thus, mice established as pediatric osteoporosis models are not present. Further, there are cases where mice are too small in scale to be suitable for examination of effects of the drug on the bone mass and structure and bone growth.

On the other hand, a steroid-induced osteoporosis model with rats has not been established. Thus, in this Example, a model rat with Prednisolone 25 mg/pellet/60 days implanted under the skin of a 6-week-old female LEW/Cr1Crlj rat was employed as a pediatric steroid-induced osteoporosis model. In this model, it was confirmed that 2, 4 and 6 weeks after implantation of prednisolone, the femur BMD decreased, and the maximum stress of each of the femur and the lumber vertebra decreased.

Prednisolone 25 mg/pellet/60 days is administered at a dose of 0.42 mg a day. This dose is equivalent to 3.5 mg/kg/day for a 6-week-old rat (body weight: 120 g), and equivalent to 105 mg/day for a child having a body weight of 30 kg.

### (3) Experimental group (n=10 in each group)

(i) Sham group: Sham-operation + Vehicle (PBS) (subcutaneous administration)
(ii) GC group: Prednisolone (PSL) pellet 25 mg/pellet/60 days-subcutaneous implantation (GC) + Vehicle (PBS) (subcutaneous administration)
(iii) GC+Siglec-15Ab group: GC treatment was performed, and an anti-Siglec-15 antibody was subcutaneously administered once every 3 weeks at a dosage of 1 mg/kg (low dosage) or at a dosage of 10 mg/kg (high dosage). The #32A1 antibody was used as the anti-Siglec-15 antibody.
(iv) GC+ALN group: GC treatment was performed, and ALN was subcutaneously administered twice a week at a dosage of 0.014 mg/kg (low dosage) or at a dosage of 0.140 mg/kg (high dosage).

The PSL-subcutaneous implantation operation was started concurrently with administration of the anti-Siglec-15 antibody or ALN.

The dose of each drug was adjusted on the basis of the result of body weight measurement performed once a week.

### (4) Administration and observation period

6 weeks after the start of administration (age of 6 weeks to age of 12 weeks). After the end of the period (age of 12 weeks), the animals were euthanized, and evaluation was performed.

The rats of each experimental group were bred with normal feedstuff in a SPF environment. The rats were allowed to freely access feed and water.

For bone labeling, tetracycline was administered 5 days before euthanasia, and calcein was administered 2 days before euthanasia (at an interval of 3 days). The animals were killed 36 hours after calcein administration. The tetracycline was dissolved in PBS at a concentration of 10 mg/ml, and subcutaneously injected to each animal at a dosage of 25 mg/kg. The calcein was dissolved in a 1.4% sodium bicarbonate solution at a concentration of 10 mg/ml, and subcutaneously injected to each animal at a dosage of 10 mg/kg.

### (5) Evaluation items

### <Longitudinal evaluation>

### (i) Head trunk length and body weight

The head trunk length was measured 0, 3 and 6 weeks after administration. The body weight was measured once a week.

### (ii) Femur length

The femur length was measured under anesthesia every 3 weeks by micro-CT imaging.

### (iii) Bone formation marker and bone resorption marker

Before the start of administration, and 6 weeks after administration and before euthanasia, blood was collected from the tail vein, and the values of a bone formation marker (serum osteocalcin) and a bone resorption marker (serum TRACP-5b) in the blood were measured by an ELISA method.

Various operations were carried out in accordance with the schedule described in Figure 7.

### <Evaluation after isolation of specimens>

The animals were euthanized, and then dissected to extract the femur, the tibia and the fifth lumber vertebra as evaluation samples.

### (i) Bone morphometry

Micro-CT imaging of the femur and the tibia was performed, and the right femur length was measured.

### (ii) Histological studies

Non-decalcified hard tissue sample: a coronal section tissue of proximal left tibia 1/2 (length: about 1.5 cm) was used. The tissue was immersed and fixed in 70% ethanol, and then stored in a cool and dark place. The non-decalcified hard tissue sample was stained by Villanueva staining and was used for bright-field observation and fluorescent observation, and quantitative bone morphometry.

Decalcified tissue sample: knee joint disarticulation was performed, and samples of a coronal section tissue of the proximal tibia (proximal right tibia 1/2) and a fifth lumber vertebra coronal section were prepared.

Regarding growth disorder, there was the possibility that administration over a period of 6 weeks would not cause a change leading to generation of a difference in femur length, and therefore the growth cartilage plate width and the longitudinal growth rate were histologically measured using the non-decalcified tissue and the decalcified tissue to evaluate growth disorder ("Modern Bone Histomorphometry", 2014, WENET, Inc.).

### (iii) Mechanical test

The shaft of the left femur was subjected to a three-point bending test, and the third lumber vertebra and the distal left femur end were subjected to a compression test to evaluate the maximum stress, stiffness, elastic modulus and toughness.

### (iv) Measurement of bone mineral density

BMD and BMC measurements of the lumber vertebra and the distal left femur were performed by a DXA method using a bone densitometer (manufactured by Hitachi Medical, Ltd.).

### II. Experimental results

### (1) Effect of drug on growth

Figure 8 shows the results of longitudinally measuring the body weight, the head trunk length and the femur length after the start of the administration and observation period. In the GC group, the GC+Siglec-15 Ab group and GC+ALN group, the peak of decrease in body weight was at the age of 8 weeks, and thereafter the body weight was gradually recovered and increased (Figure 8A(i)). The head trunk length and the femur length tended to increase like the body weight over a period from the age of 9 weeks to the age of 12 weeks (Figures 8A(ii) and 8A(iii)).

Regarding the amount of change in body weight, head trunk length and femur length from the age of 6 weeks to the age of 12 weeks, there was a significant decrease for the GC group as compared to the Sham group, and there was no significant difference between the GC group and the GC+Siglec-15 Ab group and GC+ALN group (Figure 8B).

### (2) Effect of drug on bone metabolism (bone formation marker and bone resorption marker)

Figure 9 shows the results of measuring a bone formation marker (serum osteocalcin) and a bone resorption marker (serum TRACP-5b) in a blood sample collected before the start of the administration and observation period and after 6 weeks.

In the GC group, the level of TRACP-5b as the bone resorption marker increased by 75% after 6 weeks (at the age of 12 weeks) (Figures 9A and 9B).

In contrast, in the GC+Siglec-15 Ab group and the GC+ALN group, the serum TRACP-5b level significantly decreased (Figures 9A and 9B).

On the other hand, the level of osteocalcin as the bone formation marker decreased by about 27% over a period from the age of 6 weeks to the age of 12 weeks even in the Sham group (Figures 9A and 9B). The serum osteocalcin level in GC group tended to slightly decrease, but was not significantly different from the serum osteocalcin level in each of the Sham group, the GC+Siglec-15 Ab group and the GC+ALN group (Figures 9A and 9B) .

### (3) Effect of drug on primary spongiosa region

Figures 10-1 and 10-2 show the results of histologically evaluating the effect of the drug on growth for the proximal tibia at the end of the administration and observation period (at the age of 12 weeks). Figure 10-1(A) shows coronal cross-section photographs of images (3D-CT images) obtained by performing micro-CT imaging of the proximal tibia, and three-dimensionally reconstructing the acquired data. Observation of the 3D-CT images revealed that as compared to the Sham group, the GC+Siglec-15 Ab group did not show a significant change in shape, and the GC+ALN group (high dosage) had a cup-like (cupping) shape rounded as going from the epiphysis to the metaphysis (the normal shape is a trumpet-like shape).

For examining the effect of each drug on the primary spongiosa region in further detail, histological studies were conducted.

A non-decalcified tissue sample labeled with tetracycline 5 days before euthanasia and with calcein 2 days before euthanasia was observed with a fluorescence microscope (Figure 10-1(B)) to evaluate the growth rate (Figure 10-2(F)). In Figure 10-1(B), the part labeled in parallel to the growth cartilage and at a distal position represents the region labeled 2 days before euthanasia (white lower arrow), and the part labeled in parallel and at a further distal position represents the region labeled 5 days before euthanasia (white upper arrowhead). The bone growth rate was evaluated on the basis of the distance between the two labeled regions.

The results showed that there was no significant difference among the Sham group, the GC group, the GC+Siglec-15 Ab group and the GC+ALN group (Figure 10-2(F)).

Figure 10-1(C) shows the results of observing the growth cartilage and a primary spongiosa region immediately below the growth cartilage using a safranine O-stained sample (stained with acidic mucopolysaccharide) which is used for evaluation of the cartilage. As compared to the Sham group, the GC group and the GC+Siglec-15 Ab group, the GC+ALN group had more safranine O staining positive (red) regions (cartilage matrix) in the bone in the primary spongiosa region, with the safranine O staining positive regions being widely distributed to a distal position. The growth cartilage width in GC+ALN group tended to slightly decrease, but was not statistically significantly different from the growth cartilage width in each of the Sham group, the GC group and the GC+Siglec-15 Ab group (Figure 10-2(E)).

Further, Figure 10-2(D) shows the results of observing a TRACP-stained sample for evaluating bone resorption in the primary spongiosa region. The number of TRACP positive cells in the primary spongiosa region increased in the GC group as compared to the Sham group. The GC+Siglec-15 Ab group was comparable in the number of TRACP positive cells to the GC group. On the other hand, the number of TRACP positive cells significantly decreased in the GC+ALN group as compared to the Sham group and the GC group. Further, the ratio of the osteoclast surface to the bone surface (Oc. Pm/B. Pm (%)) in the primary spongiosa region was quantitatively evaluated, and the result showed that there was no significant difference between the GC+Siglec-15 Ab group and the GC group, and there was a significant decrease for the ALN administration group as compared to the GC group (Figure 10-2(C)).

### (4) Effect of drug on secondary spongiosa

Figures 11-1 and 11-2 show the results of histologically evaluating the effect of the drug on growth for the distal femur metaphysis at the end of the administration and observation period (at the age of 12 weeks). Figure 11-1(A) shows coronal cross-section photographs of images (3D-CT images) obtained by performing micro-CT imaging of the distal femur metaphysis. Observation of the 3D-CT images revealed that as compared to the bone mass in the Sham group, the bone mass in the GC group increased in a region immediately above a growth plate, and decreased in a secondary spongiosa region (within the frame) extending from the growth plate toward the proximal region (Figure 11-1(A)).

In both the GC+Siglec-15 Ab group and the GC+ALN group, the bone mass increased in a dosage-dependent manner in a region extending the region immediately above the growth plate to the proximal femur. Further, interestingly, a region where the bone mass increased extended to a point closer to the proximal position in the GC+Siglec-15 Ab group as compared to the GC+ALN group. These results indicate that longitudinal growth is hindered by administration of ALN.

Further, with the secondary spongiosa region (within the frame) as a region of interest, bone microstructure analysis was performed to quantitatively evaluate a change in bone mass (Figure 11-1(B)). The bone mass (BV/TV (%)) tended to decrease in the GC group as compared to the Sham group, and significantly increased in the GC+Siglec-15 Ab group and the GC+ALN group (high dosage) as compared to the GC group. The GC group was not significantly different in bone trabecula thickness (Tb. Th (µm)) from the Sham group, and the GC+Siglec-15 Ab group was significantly increased in the number of bone trabeculae (Tb. N (N/mm)) from the GC group. In measurement of the bone mineral density at the same part using a DXA method, the BMD of the distal femur decreased in the GC group as compared to the Sham group, and increased in a dosage-dependent manner in the GC+Siglec-15 Ab group and the GC+ALN group as compared to the GC group (Figure 11-1(C)).

Further, the distal femur end part was subjected to a compression test to evaluate the mechanical strength of the distal femur end part (Figure 11-2(D)). In the GC+Siglec-15 Ab group, the maximum stress and toughness increased in a dosage-dependent manner. The maximum stress and toughness in the GC+ALN group tended to increase, and were not significantly different from the maximum stress and toughness in the GC group. The reason why there was a difference in mechanical strength between the GC+Siglec-15 Ab group and the GC+ALN group in the femur metaphysis compression test may be that in the Siglec-15 Ab group, the bone mass increased over a wide range up to a region close to a proximal position, whereas in the GC+ALN group, the bone mass increased over a narrow range.

### (5) Conclusions

The above results show that the number of osteoclasts acting on remodeling of the secondary spongiosa was significantly decreased by any of the drugs: the anti-Siglec-15 antibody and bisphosphonate, resulting in increasing effect on bone mass. What is particularly interesting is that the bone mass increased over a wider range in a longitudinal direction in treatment with the anti-Siglec-15 antibody as compared to treatment with bisphosphonate. In growing children, the primary spongiosa formed immediately below the growth plate is gradually modeled into the secondary spongiosa, and pushed out to move toward the diaphysis. It is considered that in treatment with bisphosphonate, the movement of the trabecular bone is delayed, whereas in treatment with the anti-Seglec-15 antibody, such a delay does not occur, and therefore the bone mass efficiently increases over a wide range. Consequently, it is indicated that in pediatric osteoporosis, the anti-Siglec-15 antibody exhibits a bone mass increasing effect equal to or greater than that of bisphosphonate, and is useful in treatment of the disease.

Regarding bone growth disorder, the experiments using growing healthy rats in "A. Evaluation using growing healthy rats" above showed that administration of bisphosphonate caused long bone growth disorder, whereas administration of the anti-Siglec-15 antibody did not cause the disorder. Further, from a histological point of view, it was confirmed that administration of bisphosphonate significantly decreased the number of osteoclasts acting on resorption of the growth plate cartilage and modeling of the primary spongiosa and the cortical bone of the metaphysis, whereas administration of the anti-Siglec-15 antibody did not decrease the number of such osteoclasts. These results indicate that bisphosphonate may have a negative effect on modeling of tissues in the vicinity of the growth plate. The experiments using pediatric steroid-induced osteoporosis model rats in "B. Evaluation using pediatric steroid-induced osteoporosis model rats" above showed that administration of bisphosphonate caused abnormal bone morphology in the vicinity of metaphysis, but did not cause significant growth disorder in longitudinal growth. This may be because in these osteoporosis model rats, steroid causes severe growth disorder, and in contrast to this effect, the effect of bisphosphonate is small and unnoticeable. In fact, in the bisphosphonate high-dosage administration group, both the head trunk length and the femur length tended to decrease.

Abnormal bone morphology in the vicinity of the metaphysis due to administration of bisphosphonate has been reported in actual cases of use of bisphosphonate with human children, and the effect of long-term use of bisphosphonate is a concern (Michael P, et al. N Engl J Med, 2003). Little data has been obtained to date on the effect and safety about long-term use of bisphosphonate for pediatric steroid-induced osteoporosis, and therefore abundance of caution is necessary for use of bisphosphonate (Cochrane Database Syst Rev. 2007 Oct 17; (4): CD005324., Marini JC. Nat Rev Endocrinol. 2009 May; 5(5): 241-3.; US National Institute of Health (https://www.bones.nih.gov/health-info/bone/bone-health/juvenile/juvenile-osteoporosis#a)).

On the other hand, the above-described results show that a compensatory mechanism of Siglec-15 is present in the vicinity of the bone growth plate, and treatment with the anti-Siglec-15 antibody suppresses osteoclasts acting on remodeling in the secondary spongiosa, but does not suppress osteoclasts acting on modeling involved in growth of bones. Therefore, the anti-Siglec-15 antibody therapy is a very purposeful treatment method which can be relatively safely used for pediatric osteoporosis patients, and does not cause growth disorder, abnormal bone structure, abnormal bone quality and the like.

## Claims

1. A pharmaceutical composition for the treatment and/or prophylaxis of pediatric osteoporosis, the pharmaceutical composition comprising an antibody or a functional fragment thereof which binds to Siglec-15 and has activity of suppressing formation of osteoclasts and/or bone resorption by osteoclasts.

2. The pharmaceutical composition according to claim 1, which does not cause growth disorder, abnormal bone structure and/or abnormal bone quality.

3. The pharmaceutical composition according to claim 1 or 2, wherein the pediatric osteoporosis is pediatric osteoporosis developed due to drug administration.

4. The pharmaceutical composition according to claim 1 or 2, wherein the pediatric osteoporosis is pediatric steroid-induced osteoporosis.

5. The pharmaceutical composition according to any one of claims 1 to 4, wherein the antibody is a monoclonal antibody.

6. The pharmaceutical composition according to any one of claims 1 to 4, wherein the antibody consists of a heavy chain containing CDRH1 consisting of the amino acid sequence set forth as SEQ ID NO: 12 in Sequence Listing, CDRH2 consisting of the amino acid sequence set forth as SEQ ID NO: 13 in Sequence Listing and CDRH3 consisting of the amino acid sequence set forth as SEQ ID NO: 14 in Sequence Listing, and a light chain containing CDRL1 consisting of the amino acid sequence set forth as SEQ ID NO: 15 in Sequence Listing, CDRL2 consisting of the amino acid sequence set forth as SEQ ID NO: 16 in Sequence Listing and CDRL3 consisting of the amino acid sequence set forth as SEQ ID NO: 17 in Sequence Listing.

7. The pharmaceutical composition according to any one of claims 1 to 6, wherein the antibody is a chimeric antibody, a humanized antibody or a human antibody.

8. The pharmaceutical composition according to any one of claims 1 to 7, wherein the functional fragment of the antibody is Fab, F(ab')₂, Fab', Fv or scFv.
